# EUROPÄISCHE PATENTSCHRIFT

(11) **EP 1 962 668 B1**
(45) Veröffentlichungstag und Bekanntmachung des Hinweises auf die Patenterteilung: **17.06.2009**
(21) Anmeldenummer: 06819917.3
(22) Anmeldetag: 06.12.2006
(51) Int. Cl.: A61B 5/00, C12Q 1/00, G01N 33/487

(54) **SANDWICHSENSOR ZUR ERMITTLUNG EINER ANALYTKONZENTRATION**
SANDWICH SENSOR FOR THE DETERMINATION OF AN ANALYTE CONCENTRATION
CAPTEUR EN SANDWICH DESTINE A DETERMINER UNE CONCENTRATION D'ANALYTE

(30) Priorität: 19.12.2005 EP 05027755; 01.09.2006 DE 102006041343
(43) Veröffentlichungstag der Anmeldung: 03.09.2008
(73) Patentinhaber: F. Hoffmann-La Roche AG, 4070 Basel (CH); Roche Diagnostics GmbH, 68305 Mannheim (DE)
(72) Erfinder: BAINCZYK, Gregor, 68199 Mannheim (DE); KOTZAN, Holger, 68526 Ladenburg (DE)
(74) Vertreter: Hörschler, Wolfram Johannes
(86) Internationale Anmeldenummer: PCT/EP2006/069386
(87) Internationale Veröffentlichungsnummer: WO 2007/071562

(56) Entgegenhaltungen:
- WO-A-02/06788
- WO-A-20/05078424
- WO-A-20/05113790
- US-A- 5 509 410
- US-A1- 2003 042 137
- US-A1- 2003 078 484
- US-A1- 2004 074 785
- US-A1- 2004 111 017
- US-B1- 6 560 471
- US-B1- 6 695 958

## Beschreibung

### Gebiet der Erfindung:

Es wird ein implantierbarer Sensor zur Ermittlung einer Konzentration mindestens eines Analyten in einem Medium, insbesondere einem Körpergewebe und/oder einer Körperflüssigkeit, eine Vorrichtung, welche den implantierbaren Sensor verwendet, sowie ein Verfahren zur Herstellung des implantierbaren Sensors vorgeschlagen. Derartige Sensoren und/oder Vorrichtungen werden insbesondere im Bereich der Medizintechnik eingesetzt, beispielsweise um elektrochemisch eine Konzentration von Blutglucose, Triglyceriden, Laktat oder anderer Analyten zu bestimmen.

### Stand der Technik:

Die Bestimmung der Blutglucosekonzentrationen sowie eine entsprechende Medikation ist für Diabetiker ein essentieller Bestandteil des Tagesablaufs. Dabei muss die Blutglucosekonzentration schnell und einfach mehrmals am Tag (typischerweise zwei bis sieben Mal) bestimmt werden, um gegebenenfalls entsprechende medizinische Maßnahmen ergreifen zu können. In vielen Fällen erfolgt dabei eine Medikation mittels automatischer Systeme, insbesondere so genannte Insulinpumpen.

Um den Tagesablauf des Diabetikers nicht mehr als nötig einzuschränken, werden häufig entsprechend mobile Geräte eingesetzt, welche einfach zu transportieren und zu handhaben sein sollten, so dass eine Messung der Blutglucosekonzentration, beispielsweise am Arbeitsplatz oder auch in der Freizeit, problemlos erfolgen kann. Derzeit sind verschiedene mobile Geräte am Markt, welche teilweise nach unterschiedlichen Messmethoden und unter Verwendung unterschiedlicher Diagnoseverfahren funktionieren. Ein erstes Messverfahren beruht beispielsweise auf einer elektrochemischen Messmethode, wobei eine Blutprobe, welche vom Patienten beispielsweise durch Perforieren einer Hautschicht mittels einer Lanzette dem Körpergewebe entnommen wird, auf eine mit Enzymen und Mediatoren beschichtete Elektrode appliziert wird. Entsprechende Teststreifen für derartige elektrochemische Messverfahren sind beispielsweise in US 5,286,362 beschrieben. Andere bekannte Messmethoden verwenden optische Messverfahren, welche beispielsweise darauf beruhen, dass die zu detektierende Substanz (Analyt) mit bestimmten Nachweisreagentien reagieren kann, wobei eine Farbänderung des Reaktionsgemisches eintritt. Systeme zum Nachweis derartiger Farbreaktionen und somit zum Nachweis der entsprechenden Ahalyten sind beispielsweise aus CA 2,050,677 bekannt.

Die beschriebenen Nachweisverfahren beruhen also überwiegend darauf, dass ein Patient zunächst eine entsprechende Probe der zu untersuchenden Körperflüssigkeit entnimmt (wobei es sich beispielsweise um eine Blutprobe oder eine Urinprobe handelt) und diese dann entsprechend mittels der Testvorrichtung untersucht. Dieses Verfahren beinhaltet jedoch verschiedene Nachteile. So ist zum einem dieses Verfahren äußerst aufwändig und setzt mehrere Handhabungsschritte voraus. So muss beispielsweise eine Lanzette bereitgestellt und gespannt werden, anschließend mittels dieser Lanzette eine Hautschicht perforiert werden, dann ein so erzeugter Blutstropfen auf einen Teststreifen aufgetragen werden und anschließend dieser Teststreifen mittels eines entsprechenden Gerätes ausgewertet werden. Für viele Patienten, insbesondere ältere Menschen und Kinder, sind diese Handhabungsschritte oftmals nur schwer durchzuführen, da die Patienten beispielsweise in ihrer motorischen Fähigkeit und ihrem Sehvermögen eingeschränkt sind. Weiterhin lassen sich diese Verfahrensschritte nur in wenigen Fällen diskret durchführen, so dass beispielsweise ein Schutz der Privatsphäre des Patienten bei einer Messung am Arbeitsplatz nur unzureichend gewahrt ist. Auch kann eine Fehlbedienung des Messverfahrens leicht zu falschen Messwerten führen, mit teilweise fatalen Folgen einer auf den Messergebnissen aufbauenden falschen Medikation.

Aus dem Stand der Technik sind daher Systeme bekannt, welche in ein Körpergewebe implantiert werden können und welche beispielsweise kontinuierlich Messwerte liefern. So offenbart beispielsweise US 6,892,085 B2 ein gekapseltes Glucosesensorsystem, welches einen Glucosesensor und eine Schutzkapselung umfasst. Dabei sind drei Elektroden, eine Arbeitselektrode, eine Gegenelektrode und eine Referenzelektrode vorgesehen, welche auf einer Seite eines dünnen Substrats aufgebracht sind. Zur besseren Implantierbarkeit ist diese Elektrodenanordnung in eine Hohlnadel eingebracht, welche in Körpergewebe eingestochen wird.

Auch US 5,591,139 offenbart ein implantierbares Mikronadelsystem, mittels dessen für Diagnosezwecke Substanzen aus lebendem Gewebe entnommen werden können. Dabei wird ein geätztes dreidimensionales Substrat verwendet.

Insgesamt sind jedoch die aus dem Stand der Technik bekannten implantierbaren Sensoren bezüglich ihres Aufbaus und ihrer Herstellung äußerst aufwendig. Geht man davon aus, dass es sich bei diesen Sensoren um nur für kurze Zeit (typischerweise circa eine Woche) verwendbare Einwegsensoren handelt, so wird deutlich, dass die bei den aus dem Stand der Technik bekannten Sensoren eingesetzten Verfahren derartigen Anforderungen an Einwegartikel nicht gerecht werden.

So ist beispielsweise für die Herstellung des aus US 5,591,139 bekannten Sensors ein aufwändiges Mikrostrukturierungsverfahren erforderlich, insbesondere ein lithographisches Verfahren. Derartige Verfahren sind jedoch mit der Herstellung kostengünstiger Einwegartikel nicht vereinbar. Auch zur Herstellung des aus US 6,892,085 B2 bekannten Sensors sind aufwändige Strukturierungsverfahren erforderlich, da die Elektrodenpads sorgfältig strukturiert werden müssen. Angesichts der geringen Größe dieser Elektroden sind dafür ebenfalls lithographische Verfahren erforderlich, was die Kosten zur Herstellung derartiger Sensoren wiederum in die Höhe treibt.

Auch sind lithographische Verfahren, insbesondere das mit diesen Verfahren verbundene Ätzen von Metallschichten, nicht immer so zuverlässig, wie dies für die Herstellung medizintechnischer Produkte erforderlich ist. Insbesondere kann es vorkommen, dass einzelne Elektroden noch durch "Brücken" miteinander verbunden sind, so dass die Funktionalität der Sensoren aufgrund von Herstellungsproblemen leicht beeinträchtig oder sogar ganz verhindert sein kann. Ein weiterer Nachteil der aus dem Stand der Technik bekannten Sensoren, wie beispielsweise der aus der US 6,892,085 B2 und US 5,591,139 bekannten Sensoren, besteht weiterhin in der Verwendung einer Hohlnadel bzw. Kapillare. Die Sensoren sind in diesen Fällen in eine Kapillare eingebracht, welche die zu untersuchende Körperflüssigkeit zum Sensor hintransportiert. Nachteilig daran ist jedoch, dass die Kapillare einen ungehinderten Zugang der Analytlösung zu den Elektroden erschwert. Insbesondere kann es dadurch auch zu lokalen Konzentrationsverfälschungen kommen, welche dazu führen, dass Messergebnisse nicht den tatsächlichen Konzentrationsverhältnissen in der Körperflüssigkeit entsprechen. Dabei spielen auch komplexe Diffusionsvorgänge und Strömungsvorgänge in den Kapillaren eine Rolle und tragen zur Verfälschung bei.

In US 2004/0111017 A1 ist ein Beispiel eines In-vivo-Sensors dargestellt, welcher auf einem elektrochemischen Prinzip beruht und welcher zwei Elektroden auf einem Trägersubstrat aufweist. Dabei ist eine Arbeitselektrode, welche mit einer Detektorschicht für den nachzuweisenden Analyten beschichtet ist, unmittelbar auf das Trägersubstrat aufgebracht und durch eine Deckschicht abgedeckt. Auf der der Arbeitselektrode entgegengesetzten Seite der Deckschicht ist eine gemeinsame Referenz- und Gegenelektrode aufgebracht, welche mit der Arbeitselektrode überlappt, von dieser jedoch durch die (zwangsläufig elektrisch isolierend auszugestaltende) Deckschicht getrennt ist. Der Analyt gelangt über Diffusionsmechanismen von den Kanten des Sensors zur Arbeitselektrode. Alternativ kann auch die Deckschicht selbst Analyt-durchlässig ausgestaltet sein.

Die in US 2004/0111017 A1 gezeigte Sensoranordnung weist jedoch in der Praxis verschiedene Nachteile auf. Ein Nachteil besteht beispielsweise darin, dass die Deckschicht gleichzeitig zwei verschiedene Funktionen wahrnehmen muss, welche materialtechnisch nur schwer vereinbar sind. So muss diese Deckschicht zum einen genügende elektrisch isolierende Eigenschaften aufweisen, um die Arbeitselektrode und die Gegenelektrode gegeneinander zu isolieren. Dennoch muss die Deckschicht es ermöglichen, dass der nachzuweisende Analyt zumindest vom Rand her eindringen kann, um zur Arbeitselektrode zu gelangen, um dort elektrochemisch nachgewiesen werden zu können. Diese Diffusion muss in ausreichendem Maße erfolgen können, um ausreichende Ströme für eine Messung der Analytkonzentration (Signalhub) bereitstellen zu können. Die gleichzeitige Durchlässigkeit für eine Diffusion und dennoch genügende Isolationsfähigkeit stellt jedoch hohe Anforderungen an die Werkstoffeigenschaften. In US 2004/0111017 A1 wird zur Lösung dieser Problematik eine konstruktive Ausgestaltung des Sensors vorgeschlagen, bei der im Schichtaufbau Diffusionskanäle vorgesehen sind, die ein Eindringen des Analyten ermöglichen. Diese Konstruktion ist jedoch technisch derart aufwendig, dass die Fertigungsvorteile, welche ein Schichtaufbau bieten kann nahezu vollständig wieder verschenkt werden.

Dokumente US 2004/0111017 A1 und US 2003/0042137 A1 offenbaren implantierbare Sensoren entsprechend dem Oberbegriff der unabhängigen Ansprüche.

### Aufgabe der Erfindung:

Aufgabe der Erfindung ist es somit, einen Sensor zur Ermittlung einer Konzentration mindestens eines Analyten in einem Medium zur Verfügung zu stellen, welcher einfach und kostengünstig mittels eines zuverlässigen Herstellungsverfahrens herstellbar ist und welcher die Nachteile der aus dem Stand der Technik bekannten Sensoren und Verfahren nach Möglichkeit vermeidet. Insbesondere soll der Sensor implantierbar sein und ausreichende Signalhübe gewährleisten.

### Darstellung der Erfindung:

Diese Aufgabe wird durch die Erfindung mit den Merkmalen der unabhängigen Ansprüche gelöst. Vorteilhafte Weiterbildungen der Erfindung sind in den Unteransprüchen gekennzeichnet. Der Wortlaut sämtlicher Ansprüche wird hiermit durch Bezugnahme zum Inhalt der Beschreibung gemacht.

Es wird ein implantierbarer Sensor zur Ermittlung einer Konzentration mindestens eines Analyten in einem Medium, insbesondere in einem Körpergewebe und/oder einer Körperflüssigkeit, vorgeschlagen. Bei dem Analyten kann es sich beispielsweise um Glucose, Triglyceride, Laktat, Hormone oder andere Analyten handeln, welche insbesondere in der Medizin eine Rolle spielen. Alternativ oder zusätzlich kann der Sensor jedoch auch zur Messung anderer Arten von Analyten eingesetzt werden. Insbesondere beruht der Sensor auf der Verwendung eines elektrochemischen Messverfahrens.

Ein Grundgedanke der Erfindung besteht darin, die oben beschriebene Problematik, dass einerseits eine elektrische Isolierung der Elektroden gegeneinander erforderlich ist und andererseits die Elektroden für den mindestens einen Analyten möglichst frei zugänglich sein sollten, durch einen geeigneten Schichtaufbau zu lösen. Dementsprechend wird ein implantierbarer Sensor derart ausgestaltet, dass mindestens zwei Elektroden auf einem elektrisch isolierenden Trägersubstrat in mindestens zwei verschiedenen Schichtebenen, beispielsweise auf einer Vorder- und einer Rückseite und/oder in verschiedenen Stufenebenen, aufgebracht werden. Die mindestens zwei Elektroden sind durch das mindestens eine Trägersubstrat elektrisch voneinander getrennt.

Weiterhin weisen die mindestens zwei Elektroden Elektrodenflächen auf, also aktive Oberflächen, an denen elektrochemische Reaktionen (Redoxreaktionen) ablaufen können. Diese mindestens zwei Elektrodenflächen sind erfindungsgemäß, um einen maximalen Signalhub zu erzielen, bei in das Medium implantiertem Sensor dem Medium zugewandt und stehen unmittelbar oder über eine Analyt-durchlässige Membranschicht im Wesentlichen gleichförmig mit dem Medium in Kontakt. Dies bedeutet insbesondere, dass nicht nur, wie in US 2004/0111017 A1, ein Eindringen von Analyt über den Sensorrand möglich sein soll, sondem die Zugänglichkeit soll senkrecht zu den Elektrodenflächen vom Medium her erfolgen können, und zwar im Wesentlichen ungehindert über die gesamten Elektrodenflächen. Geringfügige Einschränkungen der Zugänglichkeit können dabei in Kauf genommen werden, beispielsweise durch geringfügige Abdeckungen der Elektrodenflächen, beispielsweise von nicht mehr als 10% der Elektrodenflächen.

Durch die erfindungsgemäße Ausgestaltung wurden also die beiden Funktionen, welche in US 2004/0111017 A1 durch ein und dieselbe Schicht wahrgenommen werden, nämlich die Deckschicht zwischen den sich überlappenden Elektroden, getrennt und somit optimiert. Die elektrische Isolation wird nunmehr durch das mindestens eine isolierende Trägersubstrat und die räumliche Anordnung der mindestens zwei Elektroden wahrgenommen. Die Zugänglichkeit der Elektroden für das Medium bzw. den Analyten wird dadurch gewährleistet, dass sich die mindestens zwei Elektroden nicht mehr, wie in US 2004/0111017 A1, ganz oder teilweise überlappen, sondern dass diese beispielsweise nebeneinanderliegend angeordnet sind und frei mit dem Medium in Kontakt stehen. Unter "frei" ist dabei, wie oben beschrieben, neben einer völlig freien Zugänglichkeit auch ein Kontakt über eine Membranschicht zu verstehen, welche für den mindestens einen Analyten durchlässig ist. Diese Membranschicht kann beispielsweise die Biokompatibilität und somit die Implantierbarkeit des Sensors gewährleisten (siehe unten). Diese Membranschicht, welche beispielsweise eine Diffusion von Elektrodenmaterial oder Teilen davon in das Medium (z.B. Zellgewebe) verhindern soll oder einen direkten Kontakt der mindestens einen Elektrode mit dem Medium, muss keinerlei Anforderungen an die elektrische Isolationswirkung erfüllen. Die Membranschicht kann also, solange die Biokompatibilitätsanforderungen erfüllt sind, beispielsweise beliebig dünn ausgestaltet sein. Insgesamt übernehmen also in diesem Fall die Membranschicht und das mindestens eine Trägersubstrat gemeinsam die Aufgaben, welche in US 2004/0111017 A1. die Deckschicht alleine, ggf. in Zusammenwirkung mit den komplexen Diffusionskanälen, erfüllt.

Der implantierbare Sensor kann auf verschiedene Arten vorteilhaft erfindungsgemäß weitergebildet werden. Die beschriebenen vorteilhaften Weiterbildungen können dabei einzeln oder in Kombination verwendet werden.

Vorzugsweise umfassen die mindestens zwei Elektroden mindestens eine Arbeitselektrode und mindestens eine weitere Elektrode, insbesondere mindestens eine Gegenelektrode und/oder mindestens eine Referenzelektrode. Dabei sind vorzugsweise die mindestens eine Arbeitselektrode und die mindestens eine weitere Elektrode in unterschiedlichen Schichtebenen des Schichtaufbaus angeordnet. Die Messung der Analytkonzentration erfolgt dann vorzugsweise nach Benetzung durch das Medium bzw. nach Implantierung des Sensors durch amperometrische Messung zwischen den mindestens zwei Elektroden (Arbeits- und Gegenelektrode), insbesondere mittels einer Gleichspannung. Eine Referenzelektrode zur stromlosen Messung des Arbeitselektrodenpotenzials kann zusätzlich verwendet werden.

Unter "in unterschiedlichen Schichtebenen" soll dabei insbesondere zu verstehen sein, dass zwischen den mindestens zwei Elektroden mindestens ein isolierendes Trägersubstrat angeordnet ist, so dass mindestens zwei der mindestens zwei Elektroden durch das isolierende Trägersubstrat getrennt sind. Somit wird, im Gegensatz zum oben beschriebenen Stand der Technik, bei diesem Aufbau eines implantierbaren Sensors die "dritte Dimension" mitgenutzt.

Zwar sind aus dem Stand der Technik elektrochemische Zellen, beispielsweise elektrochemische Mikrozellen, bekannt, bei welchen Elektroden auf gegenüberliegenden Seiten eines Trägers angeordnet sind. In diesem Fall handelt es sich jedoch bei dem Träger regelmäßig um ein Elektrolytmaterial, insbesondere einen Feststoffelektrolyten. Im Gegensatz dazu weist das mindestens eine isolierende Trägersubstrat des erfindungsgemäßen elektrochemischen Sensors ein elektrisch nicht leitendes Material auf. Dies soll insbesondere bedeuten, dass, wenn an zwei auf entgegengesetzten Seiten des mindestens einen isolierenden Trägersubstrats angeordnete Elektroden eine Spannung von bis zu ca. 1-2 Volt angelegt wird, Ströme von nicht mehr als einem Milliampere, vorzugsweise von nicht mehr 100 Mikroampère, und besonders bevorzugt von nicht mehr als 10 Mikroampère, fließen. Dadurch ist gewährleistet, das der Messstrom aufgrund des elektrochemischen Messverfahrens erheblich größer ist als der Strom, welcher trotz der isolierenden Eigenschaften des elektrisch nicht leitenden Materials des mindestens einen isolierenden Trägersubstrats durch das mindestens eine isolierende Trägersubstrat fließt.

Weiterhin kann das mindestens eine Trägersubstrat auch zusätzlich derart ausgestaltet sein, dass eine Diffusion des mindestens einen Analyten durch das mindestens eine Trägersubstrat nicht möglich oder stark behindert ist. Insofern unterscheidet sich das mindestens eine Trägersubstrat hinsichtlich der Materialanforderungen deutlich auch von der in US 2004/0111017 A1 verwendeten Deckschicht, so dass eine größere Anzahl geeigneter Materialien zur Verfügung steht und genutzt werden kann.

Zur Kontaktierung der mindestens zwei Elektroden kann der implantierbare Sensor weiterhin eine oder mehrere Elektrodenkontaktschichten aufweisen. Dabei kann sich beispielsweise um elektrisch leitende Schichten handeln, z. B. metallische Schichten, insbesondere um Schichten, welche Karbon, Graphit, Gold, Silber, Platin, und/oder Aluminium aufweisen. Auch mehrschichtige Aufbauten sind möglich. Auch organische Leitermaterialien kommen als elektrisch kontaktierende Elektrodenkontaktschichten in Betracht.

Da die mindestens zwei Elektroden in mindestens zwei Schichtebenen des Schichtaufbaus des Sensors angeordnet sind, ist es nunmehr möglich, dass sich die mindestens zwei Elektroden und/oder die mindestens zwei Elektrodenkontaktschichten im Wesentlichen über die gesamte Breite des mindestens einen isolierenden Trägersubstrats erstrecken. So kann das isolierende Trägersubstrat insbesondere eine Längserstreckung sowie eine Breite und eine Länge aufweisen, wobei sich die Elektrodenkontaktschichten und/oder die Elektroden im Wesentlichen in einem Rand des isolierenden Trägersubstrats zum anderen erstrecken. Unter "im Wesentlichen" kann dabei eine Abdeckung des isolierenden Trägersubstrats durch die Elektrodenkontaktschicht von mindestens 80%, vorzugsweise 95% und besonders bevorzugt von 100% verstanden werden. Eine Strukturierung der Elektroden und/oder Elektrodenkontaktschichten ist also aufgrund der Trennung durch den Aufbau in verschiedenen Ebenen nicht mehr erforderlich, so dass auf aufwändig lithographische Strukturierungsverfahren oder Laserstrukturierungsverfahren (z. B. Laserablation) verzichten werden kann. Die Vorteile dieser Möglichkeit werden unten deutlich, wenn ein mögliches Herstellungsverfahren des implantierbaren Sensors im Detail beschrieben wird.

Weiterhin kann der implantierbare Sensor mindestens eine Isolatorschicht aufweisen, welche die mindestens zwei Elektrodenkontaktschichten zumindest teilweise, vorzugsweise jedoch vollständig, gegen das Medium, insbesondere das Körpergewebe und/oder die Körperflüssigkeit, abdeckt und vorzugsweise elektrisch isoliert. Dabei können die beispielsweise mindestens zwei Elektrodenkontaktschichten teilweise von den mindestens zwei Elektroden bedeckt sein, wobei die unbedeckten Bereiche (z. B. nachträglich) durch die mindestens eine Isolatorschicht bedeckt werden. Vorzugweise werden als Isolatorschichten selbstklebende Folienschichten eingesetzt. Auf diese Weise wird beispielsweise durch die mindestens eine Isolatorschicht verhindert, dass an den unbedeckten Bereichen der Elektrodenkontaktschichten bei Kontakt mit dem umgebenden Medium und Anlegen einer Spannung unerwünschte elektrochemische Reaktionen, beispielsweise Elektrolysereaktionen unter Gasbildung, ablaufen.

Neben dem oben beschriebenen Schichtaufbau können auch noch weitere, nicht genannte Schichten vorgesehen sein. So kann es sich bei dem mindestens einen isolierenden Trägersubstrat zunächst um ein Trägersubstrat aus beispielsweise einem Kunststoff-, Keramik- oder Papiermaterial handeln. Alternativ oder zusätzlich kann das mindestens eine Trägersubstrat selbst bereits um einen mehrschichtigen Aufbau aufweisen, beispielsweise ein Substratmaterial, welches mit weiteren Schichten besichtet ist. So können beispielsweise Substratmaterialen mit zusätzlichen Barriereschichten eingesetzt werden. Besonders bevorzugt ist es, wenn das mindestens eine isolierende Trägersubstrat als Werkstoff ein Polymer, ein isolierendes Polymer, vorzugsweise einen Polyester, aufweist. Zwischen den mindestens einen isolierenden Trägersubstrat und die mindestens zwei Elektrodenkontaktschichten und/oder die mindestens zwei Elektroden können auch weitere Schichten eingebracht sein, beispielsweise leitende oder elektrisch isolierende Schichten.

Der implantierbare Sensor gemäß der obigen Beschreibung kann beispielsweise in eine Kanüle, wie aus dem Stand der Technik bekannt, eingesetzt werden. Besonders bevorzugt ist es jedoch, wenn der implantierbare Sensor unmittelbar, das heißt ohne umgebende Kanüle oder Kapillare in das Medium insbesondere das Körpergewerbe, eingebracht werden kann. Auf diese Weise ist gewährleistet, dass die in den mindestens zwei Ebenen angeordneten Elektroden frei von Körperflüssigkeit umspült werden können. Zu diesem Zweck kann der implantierbare Sensor, wie oben bereits beschrieben, mindestens eine den Schichtaufbau ganz oder teilweise umschließende Membranschicht aufweisen. Vorteilhafterweise weist diese mindestens eine Membranschicht zumindest teilweise eine Durchlässigkeit für den mindestens einen Analyten, welcher nachgewiesen werden soll, auf. Beispielsweise kann die mindestens eine Membranschicht eine Durchlässigkeit für Glucose, Laktat, Triglyceride und/oder weitere Analyten aufweisen. Dabei sollte jedoch vorteilhafterweise die mindestens eine Membranschicht undurchlässig sein für bei dem elektrochemischen Messverfahren eingesetzte Hilfschemikalien, beispielsweise für eingesetzte Enzyme, welche auf eine oder mehrere der Elektroden aufgebracht sind. Beispielsweise kann es sich dabei um Glucoseoxidase handeln. Somit gewährleistet die Membranschicht auch, dass diese Hilfschemikalien, welche teilweise von erheblicher Toxizität sind (z. B. stellt Glucoseoxidase ein Zellgift da), nicht in das Körpergewebe gelangen und da Schaden anrichten können.

Die mindestens eine Membranschicht kann beispielsweise den Bereich, in welchem die Elektroden aufgebracht sind, umschließen. Beispielsweise kann die mindestens eine Membranschicht ein Polyurethan aufweisen. Auch ein mehrschichtiger Membranschichtaufbau ist möglich. Beispielweise können für das Aufbringen des Polyurethans nasschemische Verfahren, z. B. Tauchverfahren oder Sprühverfahren, oder auch andere bekannte Beschichtungsverfahren eingesetzt werden.

Die Ausgestaltung der mindestens zwei Elektroden kann auf verschiedene Weisen erfolgen. Insbesondere können die mindestens zwei Elektroden, wie oben beschrieben, mindestens eine Arbeitselektrode und mindestens eine weitere Elektrode, welche mindestens eine Gegenelektrode und mindestens eine Referenzelektrode aufweist, umfassen. Insbesondere sollte die mindestens eine Gegenelektrode ein zu einem Redoxverhalten der mindestens einen Arbeitselektrode umgekehrtes Redoxverhalten aufweisen. Eine Gegenelektrode und eine Referenzelektrode können auch als gemeinsame Elektrode ausgebildet sein, vorzugsweise als eine gemeinsame Elektrode, deren Fläche größer ist als die Fläche der mindestens einen Arbeitselektrode. Aus dem Stand der Technik sind Beispiele für die Verwendung von Elektrodenmaterialien für elektrochemische Messverfahren bekannt. So können beispielsweise Elektroden mit Enzymen oder anderen chemischen Hilfsstoffen beschichtet werden, welche spezifisch für den nachzuweisenden Analyten sind. Beispielsweise kann zum Nachweis von Glucose Glucoseoxidase (GOD) eingesetzt werden, welche Glucose in Gluconolakton umwandelt. Die dabei freiwerdenden Ladungsträger werden nachgewiesen. Um diesen Nachweis zu ermöglichen, werden die überspannungsreduzierenden Materialien eingesetzt, welche quasi als "Ladungsvermittler" zwischen dem Medium und den Elektroden dienen.

Vieler dieser überspannungsreduzierenden Materialien sind jedoch gesundheitsschädlich. Insbesondere haben sich dabei Mediatoren als toxisch erwiesen, so dass eine Inmobilisierung dieser überspannungsreduzierenden Materialien für einen Einsatz in implantierbaren Sensoren in vielen Fällen erforderlich ist. Zur Inimobilisierung kann beispielsweise eine kovalente Bindung an die Elektrode und/oder eine Schicht der Elektrode, beispielsweise eine Metallschicht, erfolgen. Insbesondere kann diese Technik zur Immobilisierung von Mediatoren eingesetzt werden. Eine zweite Möglichkeit besteht darin, das Überspannungsreduzierende Material in eine unlösliche Schicht zu integrieren, welche unlöslich ist in der den implantierbaren Sensor im implantierten Zustand umgebende Flüssigkeit, insbesondere der Körperflüssigkeit. Dies kann beispielsweise bei der Verwendung von Braunstein erfolgen, welcher als Paste auf die Elektrode aufgetragen wird und dann nach dem Trocknen unlöslich ist.

Als Mediatoren können beispielsweise Nitroseaniline, Hexacyanoferrat, Ferrocene oder andere bekannte Mediatoren eingesetzt werden. Neben Braunstein lassen sich auch andere Materialien einsetzen.

Neben der beschriebenen Ausgestaltung der mindestens einen Arbeitselektrode kann auch die Ausgestaltung der mindestens einen Referenzelektrode und/oder der Ausgestaltung der mindestens einen Gegenelektrode auf verschiedene Weisen erfolgen. So sollte die mindestens eine Referenzelektrode ein Elektronensystem mit einem elektrochemischen Potenzial aufweisen, welches sich in einem Arbeitsbereich des implantierbaren Sensor nicht oder nur unwesentlich ändert. So sollte sich beispielsweise bei einer typischen Spannungsbelastung (also einer Spannung zwischen Arbeitselektrode und Referenzelektrode) von typischerweise 300-500 Millivolt, z.B. 450 Millivolt, das elektrochemische Potenzial der mindestens einen Referenzelektrode vorzugsweise um nicht mehr als 1 Millivolt, vorzugsweise um licht mehr als eine im 1 Mikrovolt-Bereich liegende Änderung, ändern. Auf diese Weise ist sichergestellt, dass die Referenzelektrode als wirkliche Referenz wirkt, mit deren Potenzial das elektrochemische Potentziel der mindestens einen Arbeitselektrode verglichen werden kann.

Grundsätzlich lassen sich für die Referenzelektrode eine Vielzahl von Materialien und/oder Materialkombinationen einsetzten. Also besonders vorteilhaft hat sich dabei ein Silber/Silberchlorid(Ag/AgCl)-Elektrodensystem erwiesen. Auch andere Elektrodensysteme sind prinzipiell einsetzbar, sind jedoch weniger üblich, wie z. B. HgCl₂- Elektrodensysteme.

Auch die mindestens eine Gegenelektrode kann auf eine Vielzahl von verschiedenen Weisen ausgestaltet sein. Dabei sollte jedoch gewährleistet sein, dass die mindestens eine Gegenelektrode ein zu einem Redoxverhalten der mindestens einen Arbeitselektrode umgekehrtes Redoxverhalten gegenüber der umgebenden Flüssigkeit aufweist. Wenn also an der Arbeitselektrode eine Oxidation stattfindet, sollte an der Gegenelektrode eine Reduktion stattfinden und umgekehrt. Prinzipiell lassen sich reine Metalle als Gegenelektroden einsetzen, wie beispielsweise Platin. Dies hat jedoch den Nachteil, dass an derartigen Metallelektroden typischerweise eine Gasbildung auftritt, beispielsweise eine Bildung von Wasserstoff oder Sauerstoff. Eine derartige Gasbildung ist jedoch bei implantiertem Sensor im Körpergewebe unerwünscht. Insofern ist es auch hier wiederum von Vorteil, wenn ein Elektrodensystem, insbesondere ein Redoxelektrodensystem, eingesetzt wird, bei welchem eine Gasbildung vermieden wird. Insbesondere lässt sich auch hier wieder eine Ag/AgCl-Elektrodensystem einsetzen. Dabei wird beispielsweise AgCl reduziert. Daraus ist ersichtlich, dass die Gegenelektrode im Betrieb des Sensors verbraucht wird. Ist die Gegenelektrode aufgebraucht, findet wiederum häufig eine Gasbildung statt, so dass der implantierbare Sensor im Betrieb in der Regel eine begrenzte Lebensdauer aufweist: Dementsprechend ist auch von Vorteil, wenn die mindestens eine Gegenelektrode von ihrer Fläche her erheblich größer ausgestaltet wird als die mindestens eine Arbeitselektrode.

Die Art und Weise der Aufbringung der Elektroden auf die Elektrodenkontaktschichten kann auf unterschiedliche Weise erfolgen, abhängig vom verwendeten Elektrodenmaterial. Werden beispielsweise reine Metalle als Elektrodenmaterialien eingesetzt, so lassen sich beispielsweise Folienverfahren (z. B. Laminieren) einsetzen oder nasschemische Verfahren, physikalische Aufbringungsverfahren (physical vapor deposition, PVD, z. B. Aufdampfen oder Aufsputtern) oder auch chemische Aufbringungsverfahren (chemical vapor deposition, CVD). Braunstein (MnO₂/C) lässt sich beispielsweise als Beschichtung aufbringen, beispielsweise als Pastenbeschichtung. Hierbei können unterschiedliche, dem Fachmann bekannte Beschichtungsverfahren eingesetzt werden, beispielsweise Siebdruck, Rakeln, Düsenbeschichtung oder ähnliches. Dabei kann beispielsweise ein Enzym bereits mit der Paste vermischt werden, so dass Enzym und Braunstein in einem Schritt aufgebracht werden können. Alternativ kann auch zunächst Braunstein aufgebracht werden, woraufhin in einem nachfolgenden Schritt das Enzym, beispielsweise Glucoseoxidase (GOD), z. B. aufdispensiert wird oder in einem anderen nasschemischen Schritt aufgebracht wird. Entsprechend werden auch die anderen Elektroden aufgebracht. Typische E-lektrodenschichtdicken liegen im Bereich von 10 Mikrometern, können jedoch bis in den Bereich von hundert bis einigen hundert Mikrometern liegen. Auch dünnere Elektrodenschichten sind denkbar.

Erfindungsgemäß kann der implantierbare Sensor und/oder eine Vorrichtung, welche den implantierbaren Sensor enthält, für eine kontinuierliche Ermittlung einer Konzentration mindestens eines Analyten im Körpergewebe und/oder einer Körperflüssigkeit eingesetzt werden. Unter "kontinuierlich" kann dabei beispielsweise verstanden werden, dass über einen bestimmten Messzeitraum hinweg, beispielsweise eine Woche, in regelmäßigen Abständen (z. B. alle fünf Minuten oder jede Stunde) oder auch permanent, d. h. mit einer zeitlichen Auflösung, welche lediglich durch die zeitliche Auflösung eines Messgeräts begrenzt ist, Analytkonzentrationen bestimmten werden.

Eine Problematik besteht jedoch bei einer kontinuierlichen Messung in einer möglichen Drift der Vorrichtung und/oder des Sensors über den Messzeitraum. Üblicherweise erfolgt eine kontinuierliche Messung dadurch, dass zunächst mittels eines "konventionellen" Messverfahrens (z. B. Entnahme eines Blutstropfens und Messung des Blutstropfens) eine Referenzmessung durchgeführt wird, welche dann mit dem vom implantierten Sensor gelieferten Messwert abgeglichen wird. Anschließend folgt über den Messzeitraum hinweg eine Messung unter Zugrundelegung des anfänglichen Referenzmesswertes. Ändert sich dabei jedoch die Eigenschaft des implantierbaren Sensors, beispielsweise aufgrund einer Drift eines elektrochemischen Potenzials, insbesondere des elektrochemischen Potenzials einer der mindestens einen Arbeitelektrode und/oder der mindestens einen Referenzelektrode, so ist diese Messung fehlerbehaftet und unterliegt einer Drift.

In der Praxis hat es sich dabei gezeigt, dass insbesondere die mindestens eine Membranschicht und das für die mindestens eine Arbeitselektrode verwendete Elektrodenmaterial bezüglich der Drift kritische Punkte darstellen. Die Verwendung einer Braunsteinpaste, insbesondere einer mit einem Enzym (z. B. Glucoseoxidase) vermengten Braunsteinpaste, welche aufgetragen und anschließend getrocknet wird, hat sich hierbei als vorteilhaft erwiesen, da diese Auswahl eine Drift minimiert. Auch die Verwendung der beschriebenen vorteilhaften Polyurethan-Membran minimiert die Drift zusätzlich.

Der erfindungsgemäße implantierbare Sensor kann weiterhin dahingehend weitergebildet werden, dass dieser eine Einführspitze zum Einstechen des implantierbaren Sensors in das Medium, insbesondere in ein Fettgewebe (z. B. in ein interstitielles Fettgewebe), aufweist. Dabei kann beispielsweise die oberste Hautschicht durchdrungen werden und der Sensor zumindest teilweise unter die Lederhaut geschoben werden.

Diese Einführspitze kann auf verschiedene Weise ausgestaltet sein. Wie oben erwähnt und auch aus dem Stand der Technik bekannt, können beispielsweise Kanülen eingesetzt werden. Insbesondere ist es jedoch erfindungsgemäß bevorzugt, wenn der Sensor selbst, also beispielsweise der Schichtaufbau selbst, eine derartige Einführspitze aufweist.

Die Problematik bisheriger Sensoren besteht jedoch darin, dass diese üblicherweise sehr dünn und breit ausgelegt sind. Dadurch verbiegt sich jedoch der Schichtaufbau beim Einstechen, so dass die für das Einstechen des Sensors erforderliche Kraft nicht über den Sensor übertragen werden kann, dass dieser zuvor abknickt. Der erfindungsgemäße Sensor, bei welchem die Elektrodenkontaktschichten vorzugsweise großflächig aufgebracht werden und nicht strukturiert werden müssen, ermöglicht jedoch einen Aufbau mit einem hohen Aspektverhältnis. Unter einem Aspektverhältnis soll dabei das Verhältnis zwischen der Höhe und der Breite des isolierenden Trägersubstrats und/oder des gesamten Schichtsaufbaus verstanden werden. So können beispielsweise isolierende Trägersubstrate und/oder Schichtaufbauten verwendet werden, bei welchen dieses Aspektverhältnis, welches im Folgenden als k bezeichnet wird, mindestens 0,3, vorzugsweise 0,5 und besonders bevorzugt mindestens 0,9 beträgt.

Wollte man derartige Aspektverhältnisse mit herkömmlichen Sensoren, bei welchen die Elektrodenkontaktschichten strukturiert sind, erreichen, müssten, da strukturierte Elektroden eine hohe Breite des isolierenden Trägersubstrats voraussetzen, auch sehr dicke Sensoren verwendet werden. Dies bedeutet wiederum einem hohen Querschnitt des Einstichkanals des Sensors. Durch den erfindungsgemäßen Aufbau, bei welchem ein hohes Aspektverhältnis unter gleichzeitiger Minimierung der Einstichfläche erreicht wird, wird dieser Nachteil vermieden.

Besonders bevorzugt ist es, wenn der Schichtaufbau des erfindungsgemäßen Sensors ein gestufter Schichtaufbau ist. Dabei sollten mindestens zwei isolierende Trägersubstrate vorhanden sein, wobei mindestens zwei dieser isolierenden Trägersubstrate eine Stufe bilden. Insbesondere ist es bevorzugt, wenn diese Stufe in Längserstreckungsrichtung des implantierbaren Sensors (also in Einstichrichtung) ausgebildet ist. Zu diesem Zweck kann beispielsweise einer der mindestens zwei isolierenden Trägersubstrate kürzer ausgebildet sein als ein zweiter der isolierenden Trägersubstrate, wodurch vorzugsweise an der Spitze des Sensors oder in der Nähe der Spitze eine Stufe entsteht. Dadurch ist es möglich, beispielsweise drei Elektroden auszubilden, welche in drei verschiedenen Schichtebenen vorgesehen sind. Beispielsweise kann dabei mindestens eine dieser Elektroden in der Ebene der Stufe, insbesondere an der Stufe selbst, vorgesehen sein. Auf diese Weise wird die bereits oben beschriebene Sandwichstruktur weiter in die dritte Dimension erweitert.

In der Praxis haben sich aufgrund der besonderen Eigenschaften und der besonders einfachen Herstellung insbesondere zwei Schichtaufbauten bzw. deren Kombinationen bewährt, wobei die dargestellten Schichtaufbauten jedoch gegebenenfalls um zusätzliche, im Folgenden nicht erwähnte Schichten erweitert werden können. Zum einen bietet sich ein gestufter Schichtaufbau an, bei dem mindestens zwei isolierende Trägersubstrate mindestens eine Stufe bilden. Zum anderen kann, alternativ oder zusätzlich, auch ein sogenannter "Back-to-Back"-Aufbau eingesetzt werden, bei welchem mindestens zwei Elektroden auf gegenüberliegenden Seiten des mindestens einen Trägersubstrats angeordnet sind und entgegengesetzt gerichtete, dem Medium zugewandte Elektrodenflächen aufweisen.

Insbesondere kann der gestufte Aufbau derart ausgestaltet sein, dass mindestens eine Elektrode in der Ebene der mindestens einen Stufe angeordnet ist und dass die mindestens eine in der Ebene der mindestens einen Stufe angeordnete Elektrode und mindestens eine weitere Elektrode parallele, gleichgerichtete Elektrodenflächen aufweisen. Weiterhin können zwei Stufen vorgesehen sein, wobei in den zwei Ebenen der zwei Stufen gleichgerichtete Elektroden vorgesehen sind. Unter "gleichgerichtet" ist dabei zu verstehen, dass die Elektrodenflächen, also die Flächen, welche dem Medium zugewandt sind, in dieselbe Richtung weisen.

Der gestufte Aufbau kann derart mit einem "Back-to-Back"-Aufbau kombiniert werden, dass zusätzlich zu dem beschriebenen Stufenaufbau in einer der beschriebenen Ausgestaltungen mindestens eine weitere Elektrode vorgesehen ist, welche auf einer der mindestens einen Stufe abgewandten Seite des mindestens einen Trägersubstrats angeordnet ist und mit ihrer Elektrodenfläche entgegengesetzt zur Stufe orientiert ist.

Der Back-to-Back-Aufbau kann, alleine oder in Kombination, beispielsweise dadurch realisiert werden, dass mindestens ein zwischen einer ersten Elektrodenkontaktschicht und einer zweiten Elektrodenkontaktschicht eingebettetes Trägersubstrat vorgesehen ist. Auf der dem mindestens einen Trägersubstrat abgewandten Seite der ersten Elektrodenkontaktschicht kann dann mindestens eine erste Elektrode vorgesehen sein, und auf der dem Trägersubstrat abgewandten Seite der zweiten Elektrodenkontaktschicht mindestens eine zweite Elektrode.

Weiterhin wird eine Vorrichtung zur Ermittlung einer Konzentration von mindestens einem Analyten in einem Medium, insbesondere einem Körpergewebe und/oder eine Körperflüssigkeit, vorgeschlagen. Die erfindungsgemäße Vorrichtung umfasst mindestens einen implantierbaren Sensor gemäß der obigen Beschreibung der möglichen Ausgestaltungen. Weiterhin umfasst die mindestens eine Vorrichtung mindestens eine Spannungsmessvorrichtung zur Messung einer Spannung zwischen der mindestens einen Arbeitselektrode und der mindestens einen Referenzelektrode. Weiterhin kann mindestens eine Strommessvorrichtung zur Messung eines Stroms zwischen der mindestens einen Gegenelektrode und der mindestens einen Arbeitselektrode vorgesehen sein. Zusätzlich kann die Vorrichtung weiterhin eine Regelvorrichtung umfassen, welche ausgestaltet ist, den Strom zwischen der mindesten eine Gegenelektrode und der mindestens einen Arbeitselektrode zu regeln, dergestalt, dass die zwischen der mindestens einen Arbeitselektrode und der mindestens einen Referenzelektrode gemessene Spannung gerade gleich einer vorgegebenen Sollspannung ist. Dem Fachmann sind weitere Verfahren und Vorrichtungen zur Bestimmung einer elektrochemischen Potenzialdifferenz zwischen der mindestens einen Arbeitselektrode und der mindestens einen Gegenelektrode sowie eine konkrete elektronische Ausgestaltung derartiger Schaltungen bekannt.

Der beschriebene erfindungsgemäße Sensor kann beispielsweise für eine kontinuierliche Ermittlung einer Konzentration mindestens eines Analyten im Körpergewebe und/oder einer Körperflüssigkeit eingesetzt werden. Zu diesem Zweck kann beispielsweise der erfindungsgemäße Sensor, z. B. als Bestandteil der erfindungsgemäßen Vorrichtung in einer der beschriebenen Ausgestaltungen, durch Einstechen in das Körpergewebe implantiert werden. Anschließend kann dem Sensor ein gewisse Zeit zur Verfügung gestellt werden, innerhalb derer sich im Bereich des Sensors und des umgebenen Körpergewebes ein (zumindest annäherndes) Gleichgewicht einstellt. Beispielsweise kann während dieser Zeit, welche z. B. eine Stunde betragen kann, eine Quellung einzelner oder aller Schichten des Sensors erfolgen. Anschließend kann der Patient eine Kalibrationsmessung durchführen, bei welcher, wie oben beschrieben, mittels eines konventionellen Verfahrens eine Analytkonzentration in der Körperflüssigkeit bestimmt wird, beispielsweise eine Glucosekonzentration in einem Blutstropfen. Die dabei ermittelten Daten werden der erfindungsgemäßen Vorrichtung übermittelt, beispielsweise durch manuelles Eingeben oder auch durch elektronische Datenübertragung (z. B. mittels eines Kabels oder einer drahtlosen Verbindung). Dadurch wird der Vorrichtung ein Kalibrationspunkt zur Verfugung gestellt, und die erfindungsgemäße Vorrichtung kann die eingegebenen Messwerte gegen Messwerte, welche der implantierte Sensor liefert, abgleichen. Anschließend können der implantierte Sensor und die erfindungsgemäße Vorrichtung beispielsweise über einen Zeitraum von einer Woche verwendet werden, wobei beispielsweise alle 5 Minuten oder auch ununterbrochen (siehe oben) eine Messung erfolgt. Die von der erfindungsgemäßen Vorrichtung ermittelten Messwerte können beispielsweise an den Patienten ausgegeben werden, oder sie können auch anderen Systemen zur Verfügung gestellt werden, beispielsweise Medikationssystemen. So kann beispielsweise die erfindungsgemäße Vorrichtung unmittelbar mit einer Insulinpumpe verbunden werden, welche eine Insulindosierung an die gemessenen Blutglucosekonzentrationen anpasst. Nach Ablauf der Messzeit kann die komplette Vorrichtung ausgetauscht werden, oder es kann auch lediglich der erfindungsgemäße Sensor gegen einen neuen, unverbrauchten Sensor ausgetauscht werden.

Weiterhin wird ein Verfahren zur Herstellung eines implantierbaren Sensors, insbesondere eines implantierbaren Sensors gemäß der obigen Beschreibung, welcher zur Ermittlung einer Analytkonzentration in einem Medium, insbesondere in Körpergewebe und/oder einer Körperflüssigkeit, geeignet ist, vorgeschlagen. Das Verfahren weist folgende Schritte auf, wobei die Schritte nicht notwendigerweise in der nachfolgenden wiedergegebenen Reihenfolge durchgeführt werden müssen. Auch können verschiedene Verfahrensschritte wiederholt oder parallel durchgeführt werden, und es können zusätzliche, nicht aufgeführte Verfahrensschritte durchgeführt werden.

In einem Verfahrensschritt wird ein Schichtaufbau erzeugt, insbesondere ein "Sandwich"-"Schichtaufbau" oder ein ähnlicher mehrschichtiger Aufbau gemäß der obigen Beschreibung, wobei zwei Elektrodenkontaktschichten, insbesondere zwei Metallschichten, großflächig in mindestens zwei verschiedenen Schichtebenen auf mindestens eine mindestens ein isolierendes Material umfassende Trägerfolie aufgebracht werden. Für die Metallschichten und die mindestens eine Trägerfolie lassen sich beispielsweise die oben beschriebenen Materialien einsetzten. Weiterhin werden mindestens zwei Elektroden auf die mindestens zwei Elektrodenkontaktschichten aufgebracht, wobei wiederum die oben beschriebenen Werkstoffe eingesetzt werden können. Anschließend wird mittels eines Präzisions-Schneideverfahrens der Schichtaufbau in Sensorstreifen geschnitten.

Im Gegensatz zum Stand der Technik erfolgt bei dem erfindungsgemäßen Verfahren also ein Aufbringen von Elektrodenkontaktschichten großflächig und in mindestens zwei verschiedenen Schichtebenen. Eine zusätzliche Strukturierung der mindestens zwei Elektrodenkontaktschichten erfolgt vorzugsweise nicht. Dabei lassen sich aufwändige lithographische Strukturierungsverfahren vermeiden. Dennoch sind die Elektrodenkontaktschichten und somit auch die Elektroden elektrisch voneinander getrennt, da diese in unterschiedlichen Schichtebenen angeordnet sind.

Weiterhin lassen sich mittlerweile in der Technik perfektionierte Präzisionsschneideverfahren mit Schnittbreiten (d. h. minimale Breite der durch das Präzisions-Schneideverfahren erzeugten Streifen) von vorzugsweise unter 1 mm einsetzen. Im Gegensatz zum Stand der Technik müssen jedoch diese Schnittverfahren nun nicht an den bereits strukturierten Elektrodenschichten ausgerichtet werden, was bei aus dem Stand der Technik bekannten Verfahren, bei welchen in der Regel eine lithographische Strukturierung der Elektroden erfolgt, erforderlich war. So musste bei herkömmlichen Verfahren zunächst eine Strukturierung der Elektroden erfolgen, gefolgt von einer Präzisionsausrichtung eines Schneidwerkzeug an den bereits strukturierten Elektroden, gefolgt von einem Schneideverfahren. Bei dem hier beanspruchten Verfahren kann jedoch diese anfängliche Ausrichtung unterbleiben, da der erste Schnitt aufgrund der großflächig strukturierten Elektrodenkontaktschichten nicht oder nur unwesentlich positioniert werden muss.

Bei dem erfindungsgemäßen Verfahren lassen sich verschiedene Schichttechnologien einsetzen. So lassen sich beispielsweise Laminierverfahren einsetzen, insbesondere zum schichtweisen Aufbringen der Trägerfolien, von Metallschichten und/oder Isolatorschichten (z. B. Klebefolien). Verschiedene Laminierverfahren sind dem Fachmann bekannt. Dabei lassen sich auch Rolle-zu-Rolle-Folienverfahren (englisch reel-to-reel) einsetzen. Weiterhin lassen sich, insbesondere für die Aufbringung von organischen Dünnschichten oder metallischen Dünnschichten, auch physikalische Verfahren (z. B. physical vapor deposition, PVD) und/oder chemische Verfahren (chemical vapor deposition, CVD) einsetzen und/oder nasschemische Beschichtungsverfahren. Insbesondere die beschriebenen Rolle-zu-Rolle-Folienverfahren gewährleisten, dass das erfindungsgemäße Verfahren, insbesondere zur Herstellung des erfindungsgemäßen implantierbaren Sensors, im Vergleich zu im Stand der Technik bekannten Verfahren äußerst kostengünstig und zuverlässig ist. Auf diese Weise lassen sich Trägerfolien, metallische Folien, organische Schichten und/oder Isolatorschichten, aufbringen. Insbesondere lassen sich auch die beiden oben beschriebenen, besonders bevorzugten Schichtaufbauten mit einer Trägerfolie oder zwei Trägerfolien (Stufenaufbau) auf diese Weise mittels des erfindungsgemäßen Verfahrens realisieren.

Der erfindungsgemäße implantierbare Sensor, die Vorrichtung und das erfindungsgemäße Herstellungsverfahren in einer der beschriebenen Ausgestaltungen bieten gegenüber aus dem Stand der Technik bekannten Vorrichtungen und Verfahren eine Reihe von Vorteilen, welche teilweise bereits beschrieben worden sind. Insbesondere lässt sich eine geometrische Anordnung auf zwei gegenüberliegenden Seiten auf (Vorder- und Rückseite) eines isolierenden Trägersubstrats, z. B. eines isolierenden Trägersubstrats aus Kunststoff, realisieren. Bedingt durch neue Schneideverfahren mit reduzierter Schnittbreite ist es möglich, die Elektrodenabstände in vergleichbarer Größenordnung wie bei einer Anordnung einer ebenen Fläche aufzubringen. Das elektrochemische Verhalten der erfindungsgemäßen Sensoren wird demnach durch die Anordnung auf der Vorder- und Rückseite eines Substrats nicht nachteilig beeinflusst.

Weiterhin ist das beschriebene Herstellungsverfahren äußerst kostengünstig und kann auf aufwändige lithographische Strukturierungsverfahren oder Laserablation verzichten. Die Elektrodenflächen werden allein durch Schneid- und Laminierprozesse definiert, und es lassen sich kontinuierliche, kostengünstige Fertigungsverfahren einsetzen.

Auf ein aufwändiges Positionierverfahren zur Positionierung der Einzelelektroden kann verzichtet werden. Dies ist insbesondere bei miniaturisierten Sensoren (mit daraus resultierendem kleinen, das heißt schmerzfreierem Einstichkanal) mit Breiten von unter' einem Millimeter von Vorteil. Weiterhin sind die beschriebenen Sensoren sowohl in physiologischen Lösungen mit hohem Elektrolytgehalt als auch in Lösungen mit niedrigem Elektrolytgehalt einsetzbar.

Weitere Einzelheiten und Merkmale der Erfindung ergeben sich aus den nachfolgenden Beschreibungen der Ausführungsbeispiele in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt.

Im Einzelnen zeigt:
- Figur 1: eine perspektivische schematische Darstellung eines ersten Ausführungsbeispiels eines erfindungsgemäßen implantierbaren Sensors;
- Figur 2: ein zweites Ausführungsbeispiel eines erfindungsgemäßen Sensors;
- Figur 3A: eine schematische Darstellung einer erfindungsgemäßen Vorrichtung zur Ermittlung einer Konzentration mindestens eines Analyten unter Verwendung eines Sensors gemäß Figur 1;
- Figur 3B: eine schematische Darstellung eines zweiten Ausführungsbeispiel einer Vorrichtung zur Ermittlung eines Analyten unter Verwendung eines Sensors gemäß Figur 2;
- Figur 4: einen schematischen Ablaufplan eines Herstellungsverfahrens zur Herstellung eines Sensors gemäß Figur 1;
- Figur 5: einen schematischen Ablaufplan eines Herstellungsverfahrens zur Herstellung eines Sensors gemäß Figur 2;
- Figur 6: ein drittes Ausführungsbeispiel eines implantierbaren Sensors mit einer Stufenanordnung und einer gemeinsamen Elektrode;
- Figur 7: ein viertes Ausführungsbeispiel eines implantierbaren Sensors mit zwei Stufen und drei Elektroden; und
- Figur 8: ein fünftes Ausführungsbeispiel eines implantierbaren Sensors mit einer Stufe und drei getrennten Elektroden.

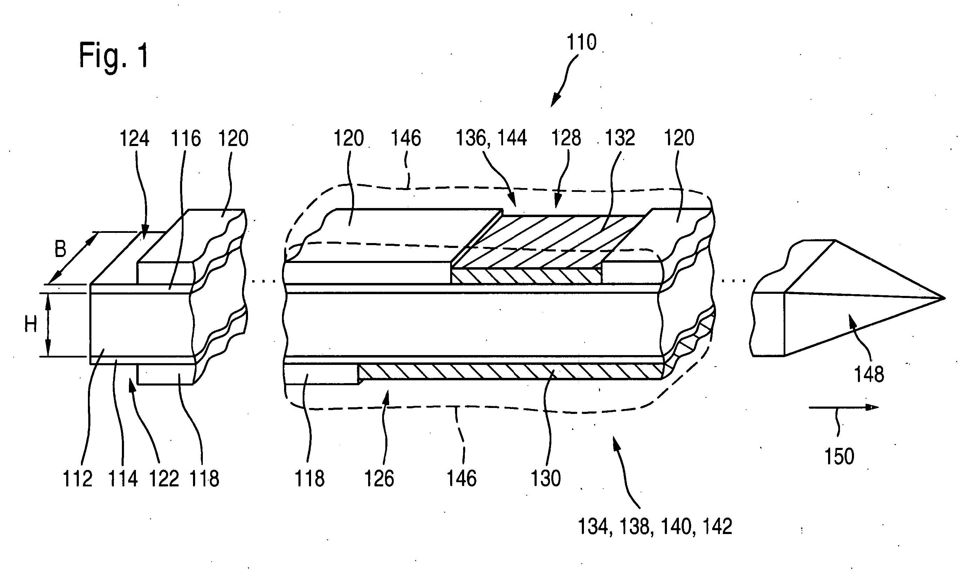

In Figur 1 ist ein mögliches erstes Ausführungsbeispiel eines erfindungsgemäßen implantierbaren Sensors 110 zur Ermittlung einer Glucosekonzentration in einer Körperflüssigkeit dargestellt. Der implantierbare Sensor 110 weist ein isolierendes Trägersubstrat 112 auf, welches in diesem Ausführungsbeispiel aus einem nicht leitenden Polyester besteht. Das isolierende Trägersubstrat 112 hat eine Höhe H von typischerweise 0,8 bis 1 Millimeter, wobei jedoch auch andere Höhen möglich sind, vorzugsweise Höhen im Bereich zwischen 0,2 und 2 Millimetern. Weiterhin weist das isolierende Trägersubstrat, 112 eine Breite B auf, welche im Bereich von einem Millimeter liegt. Somit ergibt sich in diesem Ausführungsbeispiel ein Aspektverhältnis H/B von ca. 0,8. Es sind jedoch auch andere Breiten B möglich, insbesondere Breiten zwischen 0,3 Millimetern und 2 Millimetern. Bevorzugt ist es jedoch, ein Aspektverhältnis zu wählen, welches im Bereich von 1,0 liegt, also vorzugsweise von mindestens 0,3, wobei auch das umgekehrte Aspektverhältnis (also 1/k) mindestens 0,3 betragen sollte. Diese Weite des Aspektverhältnisses gewährleistet eine hohe Stabilität des implantierbaren Sensors 110.

Das isolierende Trägersubstrat 112 ist mit einer ersten Elektrodenkontaktschicht 114 und einer auf der gegenüberliegenden Seite angeordneten zweiten Elektrodenkontaktschicht 116 beschichtet. Dabei kann es sich zum Beispiel um eine Folie oder eine andere Schicht eines Metalls wie beispielsweise Gold, Silber, Platin und/oder Aluminium handeln, welche beispielsweise auf das isolierende Trägersubstrat 112 auflaminiert ist. Die Elektrodenkontaktschichten 114, 116 sind großflächig auf das isolierende Trägersubstrat 112 aufgebracht, so dass sich diese über die volle Breite B des isolierenden Trägersubstrats 112 erstrecken.

Auf die Elektrodenkontaktschichten 114, 116 ist jeweils eine Isolatorschicht 118, 120 in Form einer in diesem Ausführungsbeispiel selbstklebenden Folienschicht aufgebracht. Diese Isolatorenschichten 118, 120 enden am linken Ende des implantierbaren Sensors 110 in Figur 1 vor dem Ende des isolierenden Trägersubstrats 112, dergestalt, das in diesem Bereich die erste Elektrodenkontaktschicht 114 und die zweite Elektrodenkontaktschicht 116 freilegen und elektrische Kontakte 122, 124 bilden. Über diese elektrischen Kontakte 122, 124 können die Elektrodenkontaktschichten 114, 116 elektrisch kontaktiert werden, beispielsweise indem Federkontakte auf diese elektrischen Kontakte 122, 124 aufgebracht werden oder mittels anderer Kontaktmittel, beispielsweise elektrischer Klemmen oder ähnlichem.

Circa ein bis zwei Zentimeter von den elektrischen Kontakten 122, 124 entfernt weisen die Isolatorschichten 118, 120 Öffnungen 126, 128 auf. Dabei ist die obere Öffnung 128 in Form eines Fensters ausgestaltet, wohingegen die untere Öffnung 126 derart ausgestaltet ist, dass hier die Isolatorschicht 118 endet. Auch andere Ausgestaltungen der Öffnungen 126, 128 sind denkbar. Im Bereich dieser Öffnungen 126, 128 sind ein erstes Elektrodensystem 130 in die Öffnung 126 und ein zweites Elektrodensystem 132 in die Öffnung 128 eingebracht, derart, dass diese Elektrodensysteme 130, 132 auf den Elektrodenkontaktschichten 114, 116 aufliegen. Die Elektrodensysteme 130, 132 bilden somit in diesen Bereichen gemeinsam mit den Elektrodenkontaktschichten 114, 116 eine erste Elektrode 134 und eine zweite Elektrode 136. Dabei setzt sich das erste Elektrodensystem 130 in dem hier dargestellten Ausführungsbeispiel aus einer Ag/AgCl-Beschichtung zusammen, wohingegen das zweite Elektrodensystem 132 eine MnO₂/C(Braunstein)-Schicht ist, vermischt mit dem Enzym Glucoseoxidase (GOD).

Die erste Elektrode 134 fungiert in diesem Ausführungsbeispiel als gemeinsame Elektrode 138 und nimmt die Funktionen von Gegenelektrode 140 und Referenzelektrode 142 wahr. Die zweite Elektrode 136 wirkt in diesem Ausführungsbeispiel als Arbeitselektrode 144.

Weiterhin ist der implantierbare Sensor im Bereich der Elektroden 134, 136 mit einer Membranschicht 146 aus Polyurethan umhüllt. Diese Membranschicht 146 ist in diesem Ausführungsbeispiel undurchlässig für das Enzym Glucoseoxidase, ist jedoch zumindest teilweise durchlässig für Glucose, wobei beispielsweise eine Diffusion der Glucose durch die Membranschicht 146 gehemmt werden kann. Mittels dieser diffusionshemmenden Wirkung kann beispielsweise eine Strombegrenzung realisiert werden.

Alternativ könnte (hier wie auch in den nachfolgenden Beispielen) auch ein Schichtaufbau realisiert werden, bei welchem lediglich die Arbeitselektrode 144, nicht hingegen die Gegenelektrode 140 und/oder die Referenzelektrode 142 mit der Glucosediffusionshemmenden Membranschicht 146 bedeckt sind. Der Vorteil einer derartigen Anordnung, bei welcher lediglich die Arbeitselektrode 144 mit der Membranschicht 146 bedeckt ist, besteht darin, dass diese Anordnung einen geringeren elektrischen Widerstand zwischen den Elektroden 140 bzw. 142 und dem umgebenden Elektrolyten aufweist. Insbesondere im Fall der Referenzelektrode 142 macht sich dieser verringerte elektrische Widerstand durch eine erhöhte Störungsfestigkeit gegenüber äußeren elektrischen Einflüssen bemerkbar.

Weiterhin ist auch ein Ausführungsbeispiel denkbar, bei welchem verschiedene Membranschichten 146 für die Elektroden 144, 142 und 140 verwendet werden oder Membranschichten, welche aus mehreren Einzelschichten mit unterschiedlicher Funktionalität zusammengesetzt sind. So könnte beispielsweise zunächst die Arbeitselektrode 144 mit einer Membranschicht 146 beschichtet werden, welche diffusionshemmende Eigenschaften für Glucose und eine Undurchlässigkeit für Glucoseoxidase aufweist, wohingegen die Gegenelektrode 140 und die Referenzelektrode 142 unbeschichtet bleiben. Anschließend könnte der implantierbare Sensor 110 (ganz oder teilweise, d.h. beispielsweise im Bereich der Elektroden 140, 142, 144) mit einer weiteren Membranschicht 146 beschichtet werden, welche biokompatible Eigenschaften aufweist, jedoch keine oder nur verringerte diffusionshemmenden Eigenschaften und welche lediglich als äußere Schutzschicht dient. In diesem Falle wäre also die Membranschicht 146 im Bereich der Arbeitselektrode 146 zweischichtig aufgebaut, im Bereich der Gegenelektrode 140 und der Referenzelektrode 142 hingegen nur einschichtig. Auch weitere Möglichkeiten der Zusammensetzung der Membranschicht 146 sind denkbar.

Weiterhin ist an den implantierbaren Sensor 110 gemäß dem Ausführungsbeispiel in Figur 1 eine Einführspitze 148 angeformt. Diese Einführspitze 148 kann beispielsweise einstückig mit dem isolierenden Trägersubstrat 112 ausgestaltet sein oder es kann sich auch um eine separat an das isolierende Trägersubstrat 112 angebrachte Einführspitze 148 handeln.

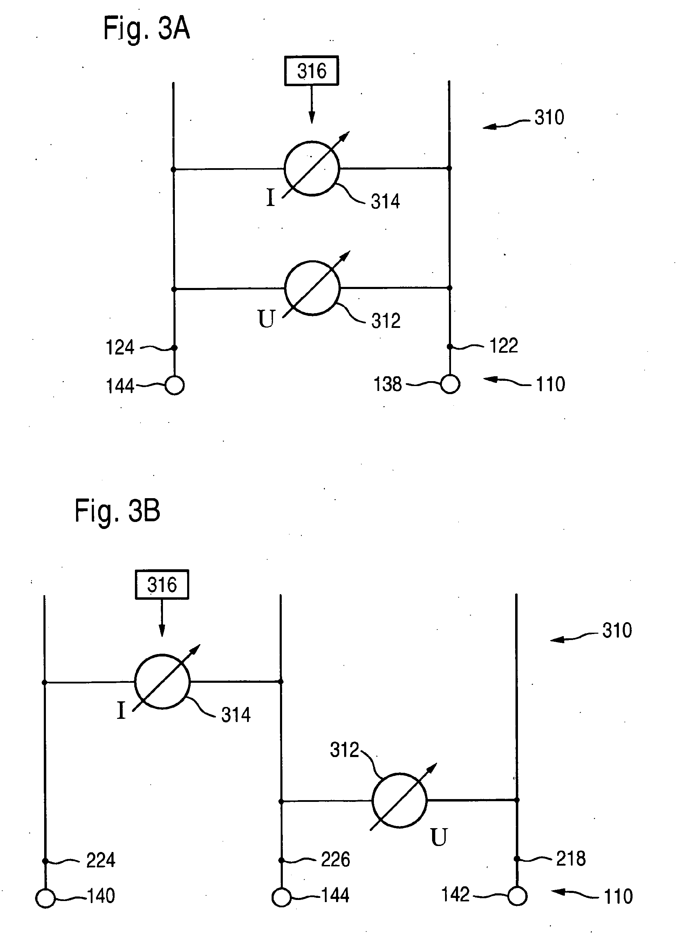

In Figur 3A ist schematisch eine Vorrichtung 310 zur Ermittlung einer Konzentration von Blutglucose unter Verwendung des Sensors 110 gemäß dem Ausführungsbeispiel in Figur 1 dargestellt. Dabei ist der Sensor 110 hier nur symbolisch durch Andeutung der Arbeitselektrode 144 und der gemeinsamen Elektrode 138 symbolisiert. Die Vorrichtung 310 weist eine Spannungsmessungsvorrichtung 312 auf, mittels derer eine elektrochemische Potenzialdifferenz (Spannung) zwischen der Arbeitselektrode 144 und der gemeinsamen Elektrode 138 gemessen werden kann. Weiterhin weist die Vorrichtung 310 eine Strommessvorrichtung 314 auf, mittels derer ein Stromfluss zwischen der Arbeitselektrode 144 und der gemeinsamen Elektrode 138 gemessen werden kann. Schließlich ist eine Regelvorrichtung 316 vorgesehen, welche den zwischen Arbeitselektrode 144 und gemeinsamer Elektrode 138 fließenden Strom derart regelt, dass die zwischen Arbeitselektrode 144 und gemeinsamer Elektrode 138 gemessene Spannung einer vorgegebenen Zollspannung entspricht. Zu diesem Zweck kann die Regelvorrichtung 316 beispielsweise eine eigene Spannungsquelle aufweisen, welche variabel ist. Aus den erforderlichen Einstellungen dieser zusätzlichen Spannungsquelle der Regelvorrichtung 316 kann dann beispielsweise auf die elektrochemische Potenzialdifferenz zwischen Arbeitselektrode 144 und gemeinsamer Elektrode 138 geschlossen werden.

Die Bestandteile der Vorrichtung 310 können räumlich getrennt voneinander sein. So kann der Sensor 110 beispielsweise teilweise oder vollständig in ein Körpergewebe implantiert werden, wohingegen die übrige Vorrichtung 310 außerhalb des Körpergewebes, beispielsweise auf der Hautoberfläche oder in einem separaten Gerät, untergebracht sind. Von der Strommessvornchtung 314 und der Spannungsmessvorrichtung 312 führen dann entsprechende Leitungen zu den elektrischen Kontakten 122, 124 des Sensors 110.

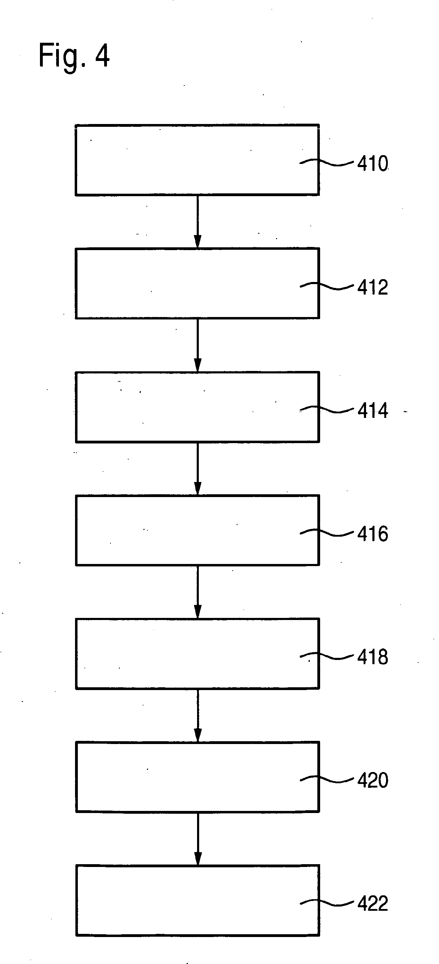

In Figur 4 ist ein Ausführungsbeispiel eines erfindungsgemäßen Verfahrens zur Herstellung eines Sensors 110 gemäß dem in Figur 1 dargestellten Ausführungsbeispiel dargestellt. Es sei darauf hingewiesen, dass jedoch auch andere Verfahren eingesetzt werden können, um den erfindungsgemäßen implantierbaren Sensor 110 gemäß der Darstellung in Figur 1 herzustellen. Auch müssen die dargestellten Verfahrensschritte nicht notwendigerweise in der angegebenen Reihenfolge durchgeführt werden.

In einem ersten Verfahrensschritt 410 werden großflächig die Elektrodenkontaktschichten 114, 116 auf eine Trägerfolie aufgebracht. Die Trägerfolie muss nicht notwendigerweise identisch sein mit den isolierenden Trägersubstraten 112, da diese isolierenden Trägersubstrate 112 aus der Trägerfolie erst durch ein späteres Schneiden hergestellt werden (siehe unten). Insofern sind sowohl Elektrodenkontaktschichten 114, 116 als auch Trägerfolie nach Durchführung dieses ersten Verfahrensschrittes 410 noch großflächig, so dass ein großflächiges "Sandwich" einer Trägerfolie, eingebettet zwischen zwei Elektrodenkontaktschichten 114, 116, entsteht. Auch die Elektrodenkontaktschichten nehmen ihre Streifenform somit erst nach dem Schneiden an. Zum Aufbringen der Elektrodenkontaktschichten 114, 116 lassen sich beispielsweise die oben beschriebenen Schichttechnologien verwenden, wobei vorzugsweise Laminiertechniken eingesetzt werden. Beispielsweise lassen sich dabei Metallfolien einsetzen, welche typischerweise Schichtdicken im Bereich von einigen 10 Mikrometern aufweisen. Es sind jedoch auch, je nach eingesetzter Schichttechnologie, dünnere oder dickere Schichten denkbar, beispielsweise Schichtdicken im Bereich von einigen 10 bis einigen 100 Nanometern bei Verwendung eines Aufdampf- oder Sputterverfahrens oder Schichtdicken im Bereich von 100 Mikrometern bei Rolle-zu-Rolle-Laminierverfahren.

In einem zweiten Verfahrensschritt, Schritt 412 in Figur 4, werden die Isolatorschichten 118, 120 aufgebracht. Dabei werden vorzugsweise selbstklebende Folien verwendet, welche wiederum großflächig auf die nach Durchführung von Schritt 410 entstandene Sandwichstruktur aufgebracht werden. Zur Erzeugung der Öffnungen 126, 128 kann beispielsweise diese selbstklebende Folie von vorneherein perforiert oder strukturiert sein, wobei jedoch eine genaue Positionierung in der Regel nicht erforderlich ist, da die genaue Positionierung der Öffnungen 126, 128 in vielen Fällen unkritisch ist. Derartige Laminierverfahren sind dem Fachmann bekannt und sind in vielfältiger Weise einsetzbar. Es lassen sich die Öffnungen 126, 128 auch nachträglich einbringen, beispielsweise durch nachträgliches Schneiden und Abziehen der Isolatorschichten 118, 120.

In einem dritten Verfahrensschritt, Schritt 414 Figur 4, wird das erste Elektrodensystem 130 aufgebracht. Dabei handelt es sich, wie oben beschrieben, um eine Ag/AgCl-Beschichtung. Zum Aufbringen dieser Ag/AgCl-Schicht kann z. B. eine Ag/AgCl-Paste, welche beispielsweise durch Vermengen von Silber und Silberchlorid-Partikeln mit einem Lösungsmittel gebildet ist, durch ein Druckverfahren (z. B. Siebdruck, Tampondruck, Schablonendruck) und/oder ein sonstiges Beschichtungsverfahren (z. B. Rakeln, Verdüsung, insbesondere durch Schlitzdüsen, Roll-Coating, o. ä.) in die Öffnung 126 eingebracht werden. Vorzugsweise wird dabei die Öffnung 126 vollständig bedeckt. Auch andere Druckverfahren lassen sich einsetzen. Es sei darauf hingewiesen, dass der in Figur 1 dargestellte Fall einen "Idealfall" darstellt, welcher in der Praxis nicht notwendigerweise in dieser Form auftreten muss. So kann beispielsweise das erste Elektrodensystem 130 auch mit der ersten Isolatorschicht 118 überlappen, was die Funktionalität der gemeinsamen Elektrode 138 nicht stört. Eine genaue Positionierung ist somit nicht erforderlich. Es kann sogar die erste Isolatorschicht 118 über einen großen Bereich mitbeschichtet werden.

Anschließend wird in Schritt 416 das zweite Elektrodensystem 132 aufgebracht. Hierbei wird, wie oben beschrieben, eine Mischung aus Braunstein und Glucoseoxidase verwendet. Dabei kann zum Aufbringen dasselbe Verfahren wie in Verfahrensschritt 414 eingesetzt werden, beispielsweise wiederum ein Druckverfahren und/oder sonstiges Beschichtungsverfahren. Auch andere Verfahren sind denkbar. Wiederum wird eine Paste eingesetzt, welche nach einer entsprechenden Trocknung fest ist und somit unlöslich ist in der umgebenden Körperflüssigkeit (Elektrolyt). Anstelle einer Mischung aus Braunstein und Glucoseoxidase kann auch zunächst eine reine Braunsteinpaste verwendet werden, auf welche dann nach Trocknung beispielsweise Glucoseoxidase aufdispensiert wird.

Anschließend wird in Verfahrensschritt 418 der bislang großflächige Schichtaufbau durch ein Präzisionsschneideverfahren hergestellt, wodurch Streifen der Breite B (vergleiche Figur 1) zu entstehen. Diese Streifen haben eine Längserstreckung parallel zu einer Einführrichtung 150 (vergleiche Figur 1). Eine genaue Positionierung beim Präzisionsschneiden senkrecht zu dieser Einführrichtung 150 ist nicht erforderlich, im Gegensatz zu herkömmlichen Verfahren, bei welchen strukturierte Elektroden in Einführrichtung 150 verwendet werden, zwischen denen die Schnitte exakt positioniert werden müssen.

Anschließend wird in Verfahrensschritt 420 die Einführspitze 148 an die nunmehr entstandenen isolierenden Trägersubstraten 112 angeformt. Beispielsweise können Einführspitzen 148 durch gleichzeitiges Schmelzen und Ziehen erzeugt werden, oder es kann auch eine entsprechende Heißformung erfolgen. Auch können alternativ oder zusätzlich separate Spitzen an die isolierenden Trägersubstrate 112 angeformt werden, beispielsweise durch Verschmelzen mit den isolierenden Trägersubstraten 112. Verschiedene Möglichkeiten sind denkbar. Alternativ kann dieser Verfahrensschritt jedoch auch weggelassen werden, da, wie oben erwähnt, der implantierbare Sensor 110 auch beispielsweise in eine separate Einführnadel eingefügt werden kann. Bevorzugt ist jedoch, wenn der Sensor 110 über eine eigene Einführspitze 148 verfügt, so dass die Elektroden 134, 136 frei von der Körperflüssigkeit (Elektrolyt) umspült werden.

Anschließend wird im letzten Verfahrensschritt, 422 in Figur 4, die Membranschicht 146 auf den Sensor 110 aufgebracht. Dabei kann beispielsweise ein einfaches Tauchverfahren verwendet werden, bei welchem der Sensor 110 (ohne Membranschicht) in eine Lösung oder eine andere das Membranmaterial (oder ein Vorprodukt desselben) enthaltende Flüssigkeit eingetaucht wird. Nach dem Tauchen kann optional zusätzlich durch Spincoating ein gleichmäßiger Flüssigkeitsfilm erzeugt werden, der anschließend getrocknet werden kann. Anschließend wird ein Trocknungsschritt durchgeführt, bei welchem die Membranschicht 146 trocknet. Eine genaue Positionierung des Tauchvorganges ist nicht erforderlich, da lediglich die Elektroden 134, 136 bedeckt werden müssen und eine darüber hinausgehende Bedeckung des Sensors 110 unerheblich für die Messergebnisse ist. Wie oben beschrieben, lassen sich beispielsweise Polyurethane als Materialien für die Membranschicht 146 einsetzen. Es lassen sich auch andere Verfahren zum Aufbringen einsetzen, beispielsweise Verfahren, bei welchen eine Polymerisierung erst nach dem Eintauchen und beim Trocknen erfolgt, oder Verfahren wie Sprühverfahren oder Druckverfahren. Als Membranmaterialien sollten insbesondere biokompatible Materialien eingesetzt werden, das heißt Materialien, welche während der Messdauer (typischerweise eine Woche, zum Teil auch mehr, zuzüglich einer "Sicherheitszeit") keine Reaktionen mit dem umgebenden Körpergewebe und/oder der Körperflüssigkeit durchführen oder toxische Substanzen in nennenswertem Maße abgeben.

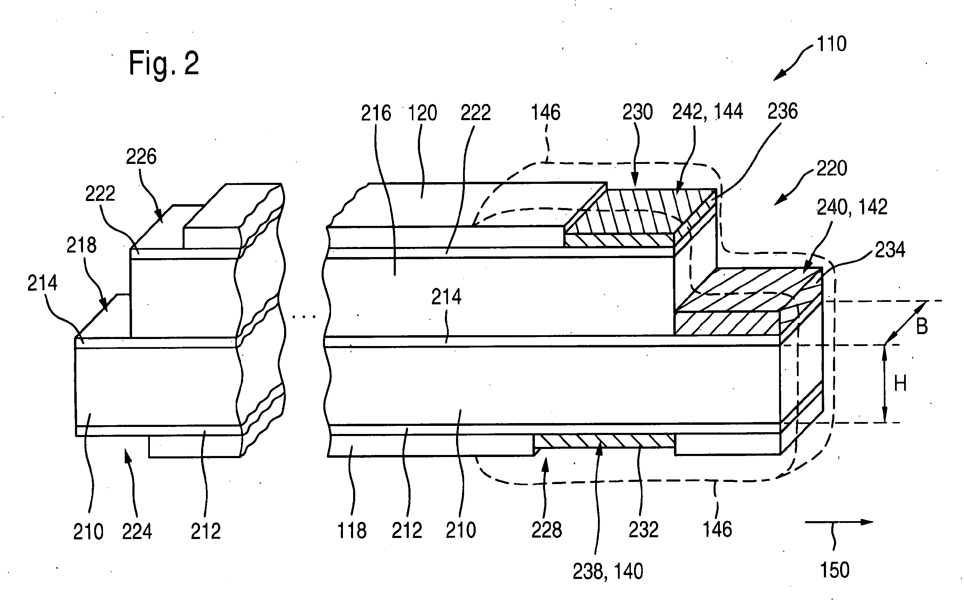

In Figur 2 ist ein zweites Ausführungsbeispiel eines erfindungsgemäßen implantierbaren Sensors 110 dargestellt, wobei in diesem Ausführungsbeispiel die Einführspitze 148 nicht dargestellt oder weggelassen ist. Der Sensor 110 gemäß dem Ausführungsbeispiel in Figur 2 weist ein erstes isolierendes Trägersubstrat 210 auf, welche zwischen einer ersten Elektrodenkontaktschicht 212 und einer zweiten Elektrodenkontaktschicht 214 eingebettet ist. An die zweite Elektrodenkontaktschicht 214 schließt sich ein zweites isolierendes Trägersubstrat 216 an, welches sich jedoch nicht über die gesamte Längserstreckung des ersten isolierenden Trägersubstrats 210 erstreckt. So ist am linken Ende des Sensors 110 (das heißt entgegengesetzt der Einführrichtung 150) ein Bereich der zweiten Elektrodenkontaktschicht 214 unbedeckt belassen, so dass sich dort ein elektrischer Kontakt zu Kontaktierung der zweiten Elektrodenkontaktschicht 214 ausbildet (zur Kontaktierung: siehe oben). Am (in Einführrichtung 150) rechten Ende endet das zweite isolierende Trägersubstrat 216 vor dem ersten isolierenden Trägersubstrat 210, so dass sich in diesem Bereich eine Stufe 220 ausbildet. Es sei hier darauf hingewiesen, dass alternativ diese Stufe 220 anstatt "nach oben" wie in Figur 2 auch "nach unten" ausgebildet sein kann, so dass insgesamt eine Invertierung des Schichtaufbaus möglich ist.

Auf der dem ersten isolierenden Trägersubstrat 210 abgewandten Seite ist das zweite isolierende Trägersubstrat 216 mit einer dritten Elektrodenkontaktschicht 222 beschichtet. Wiederum erstrecken sich sämtliche Elektrodenkontaktschichten 212, 214, 222 über die gesamte Breite B des Sensors 110. Als Materialen für die Elektrodenkontaktschichten 212, 214, 222 können grundsätzlich dieselben Materialien eingesetzt werden wie im Fall von Figur 1.

Wie auch in Figur 1 ist auch der Sensor 110 gemäß Figur 2 außenseitig mit Isolatorschichten 118, 120 beschichtet, welche in diesem Fall die erste Elektrodenkontaktschicht 212 und die dritte Elektrodenkontaktschicht 222 außenseitig elektrisch gegenüber dem umgebenden Elektrolyten, insbesondere der Körperflüssigkeit, isolieren. Wiederum kann es sich dabei um selbstklebende Folien handeln. Wie auch in Figur 1 enden auch im Beispiel gemäß Figur 2 die Isolatoren 118, 120 am linken Ende des Sensors 110 vor dem Ende der zugehörigen isolierenden Trägersubstrate 210 beziehungsweise 216 dergestalt, dass elektrische Kontakte 224, 226 frei bleiben, über welche die Elektrodenkontaktschichten 212 beziehungsweise 222 elektrisch kontaktiert werden können.

Analog zur Ausführung in Figur 1 sind auch im Ausführungsbeispiel gemäß Figur 2 Öffnungen 228, 230 in den Isolatorschichten 118 und 120 vorgesehen. Diese Öffnungen 228, 230, welche beispielsweise wiederum als "Fenster" und/oder als einfache freibleibende Bereiche der Elektrodenkontaktschichten 212, 222 ausgebildet sein können, können wiederum bereits beim Aufbringen der Isolatorschichten 118, 120 erzeugt werden oder können durch späteres Strukturieren erzeugt werden.

In die Öffnung 228, auf die zweite Elektrodenkontaktschicht 214 im Bereich der Stufe 220 und in die Öffnung 230 werden ein erstes Elektrodensystem 232, ein zweites Elektrodensystem 234 und ein drittes Elektrodensystem 236 ein- beziehungsweise aufgebracht. Dabei wird als erstes Elektrodensystem 232 und als zweites Elektrodensystem 234 wiederum, wie auch im Ausführungsbeispiel gemäß Figur 1, eines Ag/AgCl-Schicht verwendet. Als drittes Elektrodensystem 232 wird wiederum eine Braunstein-GOD-Schicht verwendet, analog zum zweiten Elektrodensystem 132 in Figur 1. Gemeinsam mit den zugehörigen Elektrodenkontaktschichten 212, 214 bzw. 222 bilden diese Elektrodensysteme 232, 234, 236 dann jeweils eine erste Elektrode 238, eine zweite Elektrode 240 und eine dritte Elektrode 242. Die erste Elektrode 238 wirkt in diesem Ausführungsbeispiel als Gegenelektrode 140, die zweite Elektrode 240 als Referenzelektrode 142 und die dritte Elektrode 242 als Arbeitselektrode 144. Somit sind in diesem Ausführungsbeispiel gemäß Figur 2 die drei Elektroden 238, 240, 242 allesamt in unterschiedlichen Schichtebenen des Schichtaufbaus angeordnet. Somit lassen sich diese Elektroden sehr breit (das heißt in diesem Fall über die volle Breite B) ausgestalten, sind jedoch trotzdem zuverlässig voneinander getrennt.

Weiterhin ist der Sensor 110 im Bereich der Elektroden 238, 240, 242 noch, analog zu Figur 1, von einer Membranschicht 146 umhüllt, welche analog zum Ausführungsbeispiel in Figur 1 ausgestaltet sein kann.

Bezüglich des Schichtdickenverhältnisses und des Aspektverhältnisses k=H/B sei in diesem Fall darauf hingewiesen, dass die oben genannte Bedingung nicht notwendig für die Höhe eines einzelnen isolierenden Trägersubstrats 210, 216 gelten soll, sondern vorzugsweise für die gesamte Dicke des in Figur 2 dargestellten Schichtaufbaus. Dies ergibt sich daraus, dass, um eine möglichst abknickfreie Einführung des Sensors 110 unter die Haut eines Patienten zu gewährleisten, der Sensor 110 insgesamt einen näherungsweise quadratischen Querschnitt haben sollte.

In Figur 3B ist eine Vorrichtung 310 zur Bestimmung einer Blutglucosekonzentration unter Verwendung eines Sensors 110 gemäß dem in Figur 1 dargestellten Ausführungsbeispiel dargestellt. Im Gegensatz zur Vorrichtung gemäß Figur 3A sind nunmehr die drei Elektroden 140, 144, 142 als getrennte Elektroden ausgestaltet. Alle drei Elektroden werden wiederum von dem Elektrolyten der Körperflüssigkeit umspült. An der Arbeitselektrode 144, welche wiederum Glucoseoxidase aufweist, findet wiederum eine Umwandlung von Glucose in Gluconolacton unter Bildung von Elektronen satt. Das elektrochemische Potenzial der Arbeitselektrode 144 wird also wiederum durch die Konzentration der Glucose in der Körperflüssigkeit bestimmt.

Wiederum weist die Vorrichtung 310 eine Spannungsmessungsvorrichtung 312 zur Spannung zwischen Arbeitselektrode 144 und Referenzelektrode 142 auf sowie eine Strommessvorrichtung 314 zur Messung eines zwischen der Gegenelektrode 140 und der Arbeitselektrode 144 fließenden Stroms. Weiterhin ist wiederum eine Regelvorrichtung 316 vorgesehen, welche den zwischen Gegenelektrode 140 und Arbeitselektrode 144 fließenden Strom derart regelt, dass die Spannung zwischen Arbeitselektrode 144 und Referenzelektrode 142 einen vorgegebenen Sollwert erreicht. Im Übrigen funktioniert das Verfahren wie anhand von Figur 3A beschrieben.

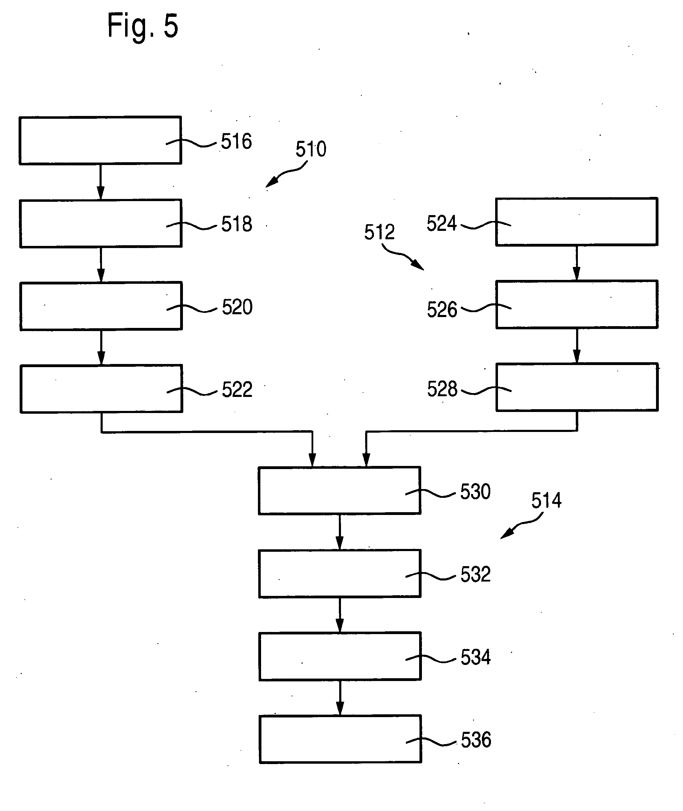

In Figur 5 ist schließlich ein erfindungsgemäßes Ausführungsbeispiel eines Herstellungsverfahrens zur Herstellung eines Sensors 110 gemäß dem Ausführungsbeispiel in Figur 2 dargestellt. Es sei jedoch wiederum darauf hingewiesen, dass auch andere Herstellungsverfahren zur Herstellung dieses Sensors 110 einsetzbar sind. Auch können wiederum die Verfahrensschritte in einer anderen Rheinfolge durchgeführt werden, und es können wiederum auch zusätzliche, nicht dargestellte Verfahrensschritte durchgeführt werden.

Anstelle eines schichtweisen Aufbaus "von unten nach oben" ist das Verfahren in Figur 5 in zwei Teilverfahren aufgeteilt, bei denen ein erster Teilschichtaufbau (Teilverfahren 510) und ein zweiter Teilschichtaufbau (Teilverfahren 512) unabhängig voneinander hergestellt werden. Anschließend werden im gemeinsamen Verfahren 514 die beiden Teilschichtaufbauten zusammengefügt und weiterverarbeitet.

Im Teilverfahren 1 wird zunächst, analog zu Verfahrensschritt 410 in Figur 4, in Schritt 516 eine erste Trägerfolie großflächig mit den Elektrodenkontaktschichten 212, 214 beschichtet. Anschließend wird auf einer Seite des so entstandenen Sandwichaufbaus in Schritt 518 die Isolatorfolie 118 auf die erste Elektrodenkontaktschicht 212 aufgebracht, analog zu Verfahrensschritt 412 gemäß Figur 4, jedoch nur einseitig.

Anschließend wird in Schritt 520 das erste Elektrodensystem 232 in die Öffnung 228 in der Isolatorschicht 118 eingebracht, beispielsweise mittels des anhand von Figur 4 beschriebennen Verfahrens.

Weiterhin wird in Schritt 522 das zweite Elektrodensystem 234 auf einen Bereich der zweiten Elektrodenkontaktschicht 214 aufgebracht, in welchem später die Stufe 220 ausgebildet ist. Alternativ kann das zweite Elektrodensystem 234 auch beispielsweise erst im Rahmen des gemeinsamen Verfahrens 514 aufgebracht werden, also nach Zusammenfügen der beiden Teilschichtaufbauten (siehe unten).

Nach Durchführung der Verfahrensschritte 516 bis 522 ist der erste Teilschichtaufbau fertig. In den Verfahrensschritten 524 bis 528 wird (insbesondere unabhängig zu den obigen Verfahrensschritten, also beispielsweise parallel) ein zweiter Teilschichtaufbau hergestellt. Dazu wird zunächst in Verfahrensschritt 524 eine zweite Trägerfolie großflächig und einseitig mit der dritten Elektrodenkontaktschicht 222 beschichtet. Anschließend wird in Verfahrensschritt 526 die Isolatorschicht 120 auf die dritte Elektrodenkontaktschicht 222 aufgebracht, wobei eine Öffnung 230 verbleibt. Für die Techniken und Materialien der Aufbringung der einzelnen Schichten sei wiederum auf das Ausführungsbeispiel gemäß Figur 4 verwiesen.

Anschließend wird im letzten Verfahrensschritt 528 des Teilverfahrens 512 das dritte Elektrodensystem 236 in die Öffnung 230 eingebracht. Damit ist das zweite Teilverfahren 512 fertig, und der zweite Teilschichtaufbau ist hergestellt.

Anschließend wird im gemeinsamen Verfahren 514 im Verfahrensschritt 530 der zweite Teilschichtaufbau, welcher sich aus der zweiten Trägerfolie, der dritten Elektrodenkoritaktschicht 222, der Isolatorschicht 120 und dem dritten Elektrodensystem 236 zusammensetzt, auf den ersten Teilschichtaufbau, welcher sich aus die Isolatorschicht 118, der ersten Elektrodenkontaktschicht 212, der ersten Trägerfolie, der zweiten Elektrodenkontaktschicht 214 und dem ersten Elektrodensystem 232 und dem zweiten Elektrodensystem 234 zusammensetzt, aufgebracht. Beispielsweise können für dieses Aufbringen wiederum Laminiertechniken verwendet werden.

Im anschließenden Verfahrensschritt 532 findet wiederum ein Präzisionsschneiden statt, bei welchem die bislang großflächigen Schichtaufbauten in streifenförmige Sensoren 110 geschnitten werden. Wiederum ist hierbei, wie oben beschrieben; eine exakte Positionierung nicht unbedingt erforderlich.

Anschließend wird in Verfahrensschritt 534 optional eine Einführspitze 148 an den Schichtaufbau angeformt, welche in der Darstellung gemäß Figur 2 nicht abgebildet ist. Schließlich wird im letzten Verfahrensschritt, Schritt 536, die Membranschicht 146 aufgebracht, analog zu Verfahrensschritt 422, beispielsweise wiederum durch ein Tauchverfahren.

Die in den Figuren 4 und 5 dargestellten beispielhaften Verfahren zur Herstellung der Sensoren 110 lassen sich gut großtechnisch realisieren. So lassen sich insbesondere Rolle-zu-Rolle-Verfahren einsetzen, für welche aus anderen Bereichen der Technik Automaten zur Verfügung stehen, so dass keine Spezialanfertigungen aufwändiger Herstellungsgeräte erforderlich ist. Das Verfahren ist äußerst kostengünstig und kann mit hoher Ausbeute und hohem Durchsatz gefahren werden.

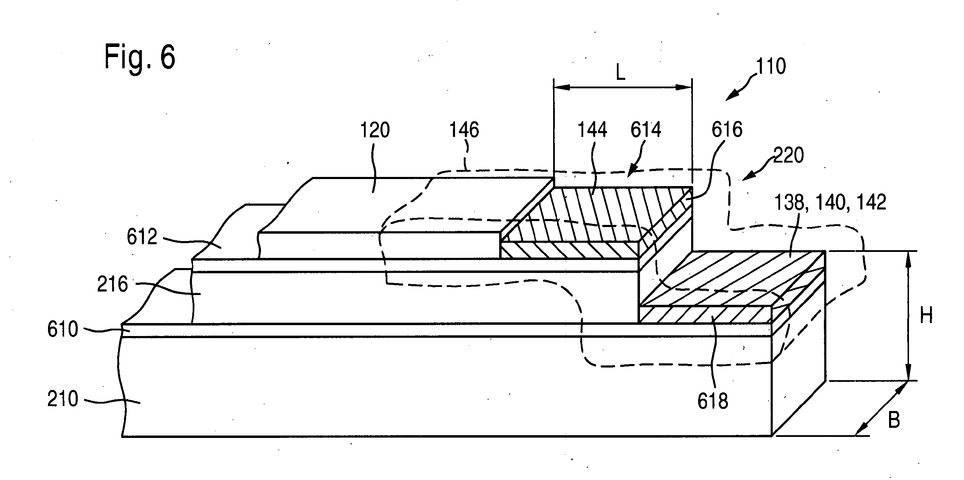

In den Figuren 6 bis 8 sind weitere vorteilhafte Ausführungsbeispiele von implantierbaren Sensoren 110 dargestellt. So zeigt Figur 6 ein Ausführungsbeispiel eines implantierbaren Sensors 110 in einer Stufenanordnung dargestellt. Wiederum weist der implantierbare Sensor 110 ein erstes isolierendes Trägersubstrat 210 auf, auf welchem eine erste Elektrodenkontaktschicht 610 aufgebracht ist. Beispielsweise kann dieses erste isolierende Trägersubstrat 210 eine Schichtdicke von ca. 200 µm aufweisen. Auf dieses erste isolierende Trägersubstrat 210 ist eine erste Elektrodenkontaktschicht 610 aufgebracht, analog zu den vorhergehenden Ausführungsbeispielen, wobei es sich beispielsweise wiederum um eine Goldschicht oder eine Schicht eines anderen Metalls oder leitfähigen Polymers handeln kann. Diese erste Elektrodenkontaktschicht hat beispielsweise eine Schichtdicke von einigen µm.

Auf die erste Elektrodenkontaktschicht 610 ist ein zweites isolierendes Trägersubstrat 216 aufgebracht, welches analog zum ersten isolierenden Trägersubstrat 210 ausgestaltet sein kann. Dabei erstreckt sich das zweite isolierende Trägersubstrat 216 elektrodenseitig, d.h. auf der rechten Seite in Figur 6, nicht über die gesamte Länge des ersten isolierenden Trägersubstrats 210, so dass in diesem Bereich eine Stufe 220 entsteht. Auf das zweite isolierende Trägersubstrat 216 ist eine zweite Elektrodenkontaktschicht 612 aufgebracht, welche analog zur ersten Elektrodenkontaktschicht 610 ausgestaltet sein kann. Auf die zweite Elektrodenkontaktschicht ist wiederum eine Isolatorschicht 120 aufgebracht, beispielsweise ein Klebeband, analog zu den vorhergehenden Ausführungsbeispielen. Diese Isolatorschicht 120 erstreckt sich elektrodenseitig (d.h. wiederum auf der rechten Seite des implantierbaren Sensors 110 in Figur 6) nicht über die gesamte Länge des zweiten isolierenden Trägersubstrats 216 und der zweiten Elektrodenkontaktschicht 612, so dass wiederum, analog zur Öffnung 230 in Figur 2, eine Öffnung 614 verbleibt, also ein Bereich, in welchem die zweite Elektrodenkontaktschicht 612 nicht von der Isolatorschicht 120 bedeckt ist.

In diesem Bereich der Öffnung 614 ist die zweite Elektrodenkontaktschicht 612 mit einem ersten Elektrodensystem 616 bedeckt, wobei es sich vorzugsweise, wie auch bei dem zweiten Elektrodensystem 132 in Figur 1, um eine MnO₂/C-(Braunstein)-Schicht, vermischt mit GOD, handelt. Insgesamt bildet sich damit im Bereich der Öffnung 614 eine Arbeitselektrode 144.

Die erste Elektrodenkontaktschicht 610, welche im Bereich der Stufe 220 nicht von dem zweiten isolierenden Trägersubstrat 216 bedeckt ist, ist in diesem Bereich von einem zweiten Elektrodensystem 618 bedeckt, welches beispielsweise analog zum ersten Elektrodensystem 130 gemäß dem Ausführungsbeispiel in Figur 1 ausgestaltet sein kann. Somit kann es sich bei dem zweiten Elektrodensystem 618 beispielsweise wiederum um eine Ag/AgCl-Beschichtung handeln. Somit bildet sich, aus dem zweiten Elektrodensystem 618 und der ersten Elektrodenkontaktschicht 610, im Bereich der Stufe 220 eine gemeinsame Elektrode 138, welche die Funktionen von Gegenelektrode 140 und Referenzelektrode 142 übernimmt. Arbeitselektrode 144 und gemeinsame Elektrode 138 sind wiederum von einer Membranschicht 146 überzogen, analog zu den vorhergehenden Ausführungsbeispielen.

Das Ausführungsbeispiel des implantierbaren Sensors 110 gemäß Figur 6 stellt somit eine Mischform der Ausführungsbeispiele gemäß den Figuren 1 und 2 dar. Zum einen ist eine Stufenanordnung gemäß dem Ausführungsbeispiel in Figur 2 vorgesehen, bei welcher beide Elektroden 144, 138 parallel angeordnet sind und mit ihren freien Elektrodenflächen in dieselbe Richtung weisen (im Ausführungsbeispiel gemäß Figur 6 nach oben). Gleichzeitig sind die Gegenelektrode 140 und die Referenzelektrode 142 als gemeinsame Elektrode 138 ausgestaltet, analog zum Ausführungsbeispiel in Figur 1. Die Breite B des implantierbaren Sensors 110 gemäß dem Ausführungsbeispiel in Figur 6 beträgt typischerweise ca. 1 mm. Damit ergibt sich, unter Berücksichtigung einer Höhe H der isolierenden Trägersubstrate 210 und 216, ein Aspekt verhältnis von ca. 0,4. Die Elektroden 144, 138 haben eine Länge L von typischerweise ca. 1 mm.

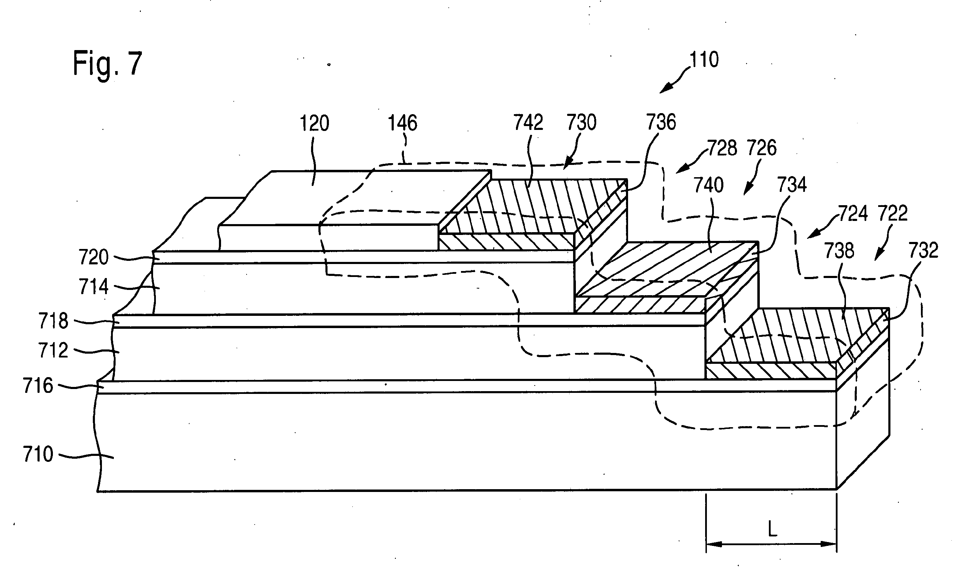

In Figur 7 ist ein weiteres Ausführungsbeispiel eines implantierbaren Sensors 110 dargestellt, welches wiederum eine Modifikation des Ausführungsbeispiels gemäß Figur 2 darstellt. Der implantierbare Sensor 110 gemäß Figur 7 weist drei isolierende Trägersubstrate 710, 712 und 714 auf, wobei diese wiederum durch zugehörige Elektrodenkontaktschichten 716, 718 und 720 bedeckt sind. Dabei sind wiederum das zweite isolierende Trägersubstrat 712 und die zweite Elektrodenkontaktschicht 718 derart auf das erste isolierende Trägersubstrat 710 und die erste Elektrodenkontaktschicht 716 aufgebracht, dass eine erste Öffnung 722 verbleibt und sich eine erste Stufe 724 bildet. Analog sind das dritte isolierende Trägersubstrat 714 und die dritte Elektrodenkontaktschicht 720 derart auf das zweite isolierende Trägersubstrat 712 und die zweite Elektrodenkontaktschicht 718 aufgebracht, dass sich eine zweite Öffnung 726 bildet, sowie eine zweite Stufe 728. Wiederum ist zudem eine Isolatorschicht 120 auf die dritte Elektrodenkontaktschicht 720 aufgebracht, wobei sich diese wiederum nicht vollständig bis zum elektrodenseitigen Ende der dritten Elektrodenkontaktschicht 720 erstreckt, so dass eine dritte Öffnung 730 verbleibt. Im Bereich der drei Öffnungen 722, 726 und 730 sind die Elektrodenkontaktschichten 716, 718 und 720 jeweils mit Elektrodensystemen 732, 734 und 736 bedeckt. Während das erste Elektrodensystem 732 und das dritte Elektrodensystem 736 wiederum Ag/AgCl-Beschichtungen beinhalten, weist das zweite Elektrodensystem 734 wiederum eine MmO₂/C-(Braunstein)-Schicht auf. Dementsprechend bilden sich eine Referenzelektrode 738, eine Arbeitselektrode 740 und eine Gegenelektrode 742 in den unterschiedlichen Schichtebenen der "Treppenstufenkonstruktion" gemäß Figur 7 aus. Somit entspricht der Schichtaufbau des Ausführungsbeispiels des implantierbaren Sensors 110 gemäß Figur 7 grundsätzlich dem Schichtaufbau in Figur 2, jedoch mit dem Unterschied, dass alle Elektroden 738, 740, 742 mit ihren Elektrodenoberflächen in eine Richtung weisen. Die Arbeitselektrode 740 ist dabei "eingerahmt" zwischen der Referenzelektrode 738 und der Gegenelektrode 742. Wiederum sind die Elektroden 738, 740, 742 umhüllt von einer Membranschicht 146. Die Schichtdicken der isolierenden Trägersubstrate 710, 712, 714 entsprechen der Schichtdicke H des isolierenden Trägersubstrats 210 gemäß Figur 6, betragen also ca. 200 µm. Die Elektrodenkontaktschichten 716, 718 und 720 weisen eine Dicke von ca. 50 µm auf, ebenso wie die Elektrodensysteme 732, 734 und 736. Die Länge L der einzelnen Öffnungen 722, 726 und 730 beträgt wiederum ca. 1 mm.

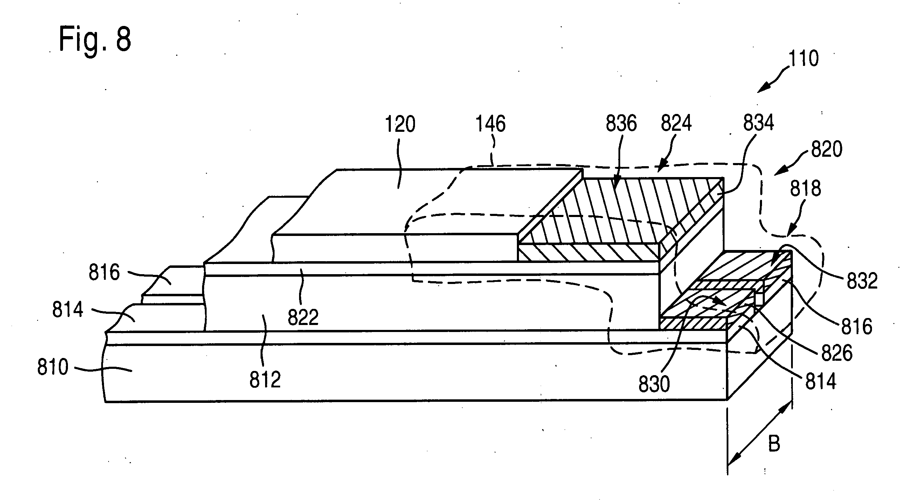

In Figur 8 ist ein weiteres Ausführungsbeispiel eines implantierbaren Sensors 110 dargestellt, welches Eigenschaften der implantierbaren Sensoren gemäß den Ausführungsbeispielen in den Figuren 6 und 7 vereinigt. So sind, analog zu Figur 6, wiederum zwei isolierende Trägersubstrate 810 und 812 vorgesehen. Auf das erste isolierende Trägersubstrat 810 sind jedoch, im Unterschied zu Figur 6, nicht eine einzelne Elektrodenkontaktschicht, sondern zwei Elektrodenkontaktschichten 814 und 816 aufgebracht, welche jeweils ungefähr die halbe Breite B des ersten isolierenden Trägersubstrats 810 einnehmen und sich entlang der Länge dieses ersten isolierenden Trägersubstrats 810 erstrecken. Dies kann fertigungstechnisch beispielsweise dadurch erfolgen, dass zunächst eine großflächige Elektrodenkontaktschicht auf das erste isolierende Trägersubstrat 810 aufgebracht wird, um anschließend mittels beispielsweise eines Schneidverfahrens oder eines Laserablationsverfahrens diese großflächige Elektrodenkontaktschicht in die beiden einzelnen Elektrodenkontaktschichten 814 und 816 elektrisch bzw. mechanisch zu trennen. Alternativ können auch beide Elektrodenkontaktschichten 814, 816 unmittelbar, d.h. bereits elektrisch voneinander isoliert auf das erste isolierende Trägersubstrat 810 aufgebracht werden. Das zweite isolierende Trägersubstrat 812 ist wiederum derart auf die beiden Elektrodenkontaktschichten 814, 816 bzw. das erste isolierende Trägersubstrat 810 aufgebracht, dass am elektrodenseitigen Ende (rechts in Figur 8) eine erste Öffnung 818 verbleibt und eine Stufe 820 sich bildet.

Auf das zweite isolierende Trägersubstrat ist eine dritte Elektrodenkontaktschicht 822 aufgebracht, welche wiederum, analog zu Figur 6, von einer Isolatorschicht 120 derart bedeckt ist, dass am elektrodenseitigen Ende (rechts in Figur 8) eine zweite Öffnung 824 verbleibt.

Im Bereich der ersten Öffnung 818 ist die erste Elektrodenkontaktschicht 814 mit einem ersten Elektrodensystem 826 bedeckt, und die zweite Elektrodenkontaktschicht 816 mit einem zweiten Elektrodensystem 828. Bei beiden Elektrodensystemen 826, 828 handelt es sich, analog zum zweiten Elektrodensystem 618 in Figur 6, um Ag/AgCl-Beschichtungen. Somit bildet sich eine Gegenelektrode 830 und eine Referenzelektrode 832, welche wiederum beispielsweise in einer Vorrichtung 310 gemäß dem Ausführungsbeispiel in Figur 3B eingesetzt werden können. In der zweiten Öffnung 824 wird wiederum eine MnO₂/C-(Braunstein)-Schicht als drittes Elektrodensystem 834 auf die dritte Elektrodenkontaktschicht 822 aufgebracht, so dass sich hier eine Arbeitselektrode 836 bildet.

Somit ist festzustellen, dass das Ausführungsbeispiel des implantierbaren Sensors 110 gemäß Figur 8 prinzipiell einen ähnlichen "1-Stufen-Aufbau" zu Figur 6 aufweist, wobei jedoch keine gemeinsame Elektrode 136 verwendet wird, sondern Gegenelektrode 830 und Referenzelektrode 832 getrennt in einer Ebene des Schichtaufbaus liegen. Dementsprechend wird beispielsweise eine Vorrichtung 310 gemäß Figur 3B eingesetzt, wohingegen für das Ausführungsbeispiel des implantierbaren Sensors 110 beispielsweise eine Vorrichtung 310 gemäß dem Ausführungsbeispiel in Figur 3A eingesetzt werden könnte. Analog ließe sich auch für das Ausführungsbeispiel in Figur 7 die Vorrichtung 310 gemäß dem Ausführungsbeispiel in Figur 3B einsetzen.

Analog zu den Ausführungsbeispielen in den Figuren 6 und 7 ist auch im Ausführungsbeispiel gemäß Figur 8 der implantierbare Sensor 110 im Bereich der Elektroden 830, 832 und 836 wieder ganz oder teilweise mit einer Membranschicht 146 überzogen. Allen drei Ausführungsbeispielen gemäß den Figuren 6 bis 8 ist gemeinsam, dass alle Elektroden mit ihrer großflächigen Elektrodenoberfläche parallel (wenn auch in unterschiedlichen Schichtebenen) liegen und in dieselbe Richtung weisen.

### Bezugszeichenliste

- 110: implantierbarer Sensor
- 112: isolierendes Trägersubstrat
- 114: erste Elektrodenkontaktschicht
- 116: zweite Elektrodenkontaktschicht
- 118: Isolatorschicht
- 120: Isolatorschicht
- 122: elektrischer Kontakt
- 124: elektrischer Kontakt
- 126: Öffnung des Isolatorschicht 118
- 128: Öffnung der Isolatorschicht 120
- 130: erstes Elektrodensystem
- 132: zweites Elektrodensystem
- 134: erste Elektrode
- 136: zweite Elektrode
- 138: gemeinsame Elektrode
- 140: Gegenelektrode
- 142: Referenzelektrode
- 144: Arbeitselektrode
- 146: Membranschicht
- 148: Einführspitze
- 150: Einführrichtung
- 210: erstes isolierendes Trägersubstrat
- 212: erste Elektrodenkontaktschicht
- 214: zweite Elektrodenkontaktschicht
- 216: zweites isolierendes Träger-Substrat
- 218: elektrischer Kontakt
- 220: Stufe
- 222: dritte Elektrodenkontaktschicht
- 224: elektrischer Kontakt
- 226: elektrischer Kontakt
- 228: Öffnung
- 230: Öffnung
- 232: erstes Elektrodensystem
- 234: zweites Elektrodensystem
- 236: drittes Elektrodensystem
- 238: erste Elektrode
- 240: zweite Elektrode
- 242: dritte Elektrode
- 310: Vorrichtung zur Ermittlung einer Blutglucosekonzentration
- 312: Spannungsmessungsvorrichtung
- 314: Strommessvorrichtung
- 316: Regelvorrichtung
- 410: Aufbringen der Elektrodenkontaktschichten
- 412: Aufbringen Isolatorschichten
- 414: Aufbringen erstes Elektrodensystem
- 416: Aufbringen zweites Elektrodensystem
- 418: Präzisionsschneideverfahren
- 420: Anformung Einführspitze
- 422: Aufbringen Membranschicht
- 510: Teilverfahren 1: Herstellung erster Teilschichtaufbau
- 512: Teilverfahren 2: Herstellung zweiter Teilschichtaufbau
- 514: gemeinsames Verfahren
- 516: Aufbringen erste und zweite Elektrodenkontaktschicht auf erste Trägerfolie
- 518: Aufbringen Isolatorfolie
- 520: Aufbringen erstes Elektrodensystem
- 522: Aufbringen zweites Elektrodensystem
- 524: Aufbringen dritte Elektrodenkontaktschicht auf zweite Trägerfolie
- 526: Aufbringen Isolatorschicht
- 528: Aufbringen drittes Elektrodensystem
- 530: Aufbringen zweiter Teilschichtaufbau auf ersten Teilschichtaufbau
- 532: Präzisionsschneiden
- 534: Anformen Einführspitze
- 536: Aufbringen Membranschicht
- 610: erste Elektrodenkontaktschicht
- 612: zweite Elektrodenkontaktschicht
- 614: Öffnung
- 616: erstes Elektrodensystem
- 618: zweites Elektrodensystem

- 710: erstes isolierendes Trägersubstrat
- 712: zweites isolierendes Trägersubstrat
- 714: drittes isolierendes Trägersubstrat
- 716: erste Elektrodenkontaktschicht
- 718: zweite Elektrodenkontaktschicht
- 720: dritte Elektrodenkontaktschicht
- 722: erste Öffnung
- 724: erste Stufe
- 726: zweite Öffnung
- 728: zweite Öffnung
- 730: dritte Öffnung
- 732: erstes Elektrodensystem
- 734: zweites Elektrodensystem
- 736: drittes Elektrodensystem
- 738: Referenzelektrode
- 740: Arbeitselektrode
- 742: Gegenelektrode

- 810: erstes isolierendes Trägersubstrat
- 812: zweites isolierendes Trägersubstrat
- 814: erste Elektrodenkontaktschicht
- 816: zweite Elektrodenkontaktschicht
- 818: erste Öffnung

- 820: Stufe

- 822: dritte Elektrodenkontaktschicht
- 824: zweite Öffnung
- 826: erstes Elektrodensystem
- 828: zweites Elektrodensystem
- 830: Gegenelektrode
- 832: Referenzelektrode
- 834: drittes Elektrodensystem
- 836: Arbeitselektrode

## Patentansprüche

1. Implantierbarer Sensor (110) zur Ermittlung einer Konzentration mindestens eines Analyten in einem Medium, insbesondere einem Körpergewebe und/oder einer Körperflüssigkeit, wobei der implantierbare Sensor (110) einen Schichtaufbau mit mindestens einem isolierenden Trägersubstrat (112; 210, 216; 710, 712, 714; 810, 812) aufweist und mindestens zwei in mindestens zwei verschiedenen Schichtebenen des implantierbaren Sensors (110) angeordneten, durch das mindestens eine isolierende Trägersubstrat (112; 210, 216; 710, 712, 714; 810, 812) elektrisch voneinander getrennte Elektroden (134, 136; 238, 240, 242; 738, 740, 742; 830, 832, 836) mit Elektrodenflächen, wobei die Elektrodenflächen bei implantierten Sensor (110) dem Medium zugewandt sind und unmittelbar oder über eine Analyt-durchlässige Membranschicht (146) großflächig und im Wesentlichen gleichförmig mit dem Medium in Kontakt stehen, wobei der implantierbare Sensor (110) weiterhin die mindestens zwei Elektroden (134, 136; 238, 240, 242; 738, 740, 742; 830, 832, 836) elektrisch kontaktierende Elektrodenkontaktschichten (114, 116; 212, 214; 222; 610, 612; 716, 718, 720; 814, 816, 822) aufweist, **dadurch gekennzeichnet, dass** das mindestens eine isolierende Trägersubstrat (112; 210, 216; 710, 712, 714; 810, 812) eine Breite aufweist, wobei sich die mindestens zwei Elektroden (134, 136; 238, 240, 242; 738, 740, 742; 830, 832, 836) und/oder die mindestens zwei Elektrodenkontaktschichten (114, 116; 212, 214; 222; 610, 612; 716, 718, 720; 814, 816, 822) über die gesamte Breite des mindestens einen isolierenden Trägersubstrats (112; 210, 216; 710, 712, 714; 810, 812) erstrecken.

2. Implantierbarer Sensor gemäß dem vorhergehenden Anspruch, **dadurch gekennzeichnet, dass** die mindestens zwei Elektroden (134, 136; 238, 240, 242; 738, 740, 742; 830, 832, 836) mindestens eine Arbeitselektrode (144; 740; 836) und mindestens eine weitere Elektrode (134, 136; 238, 240, 242; 738, 742; 830, 832), insbesondere mindestens eine Gegenelektrode (140; 742; 830) und/oder mindestens eine Referenzelektrode (142; 738; 832), umfassen, wobei die mindestens eine Arbeitselektrode (144; 740; 836) und die mindestens eine weitere Elektrode (134, 136; 238, 240, 242; 738, 742; 830, 832) in unterschiedlichen Schichtebenen des Schichtaufbaus angeordnet sind.

3. Implantierbarer Sensor (110) gemäß einem der beiden vorhergehenden Ansprüche, **dadurch gekennzeichnet, dass** die mindestens zwei Elektrodenkontaktschichten (114, 116; 212, 214; 222; 610, 612; 716, 718, 720; 814, 816, 822) zumindest teilweise, vorzugsweise vollständig, durch mindestens eine Isolatorschicht (118, 120), vorzugsweise durch mindestens eine selbstklebende Folienschicht, gegen das Medium, insbesondere das Körpergewebe und/oder die Körperflüssigkeit, abgedeckt, vorzugsweise elektrisch isoliert sind.

4. Implantierbarer Sensor (110) gemäß einem der vorhergehenden Ansprüche, **dadurch gekennzeichnet, dass** die mindestens eine Membranschicht (146) ein Polyurethan aufweist.

5. Implantierbarer Sensor (110) gemäß einem der vorhergehenden Ansprüche, **dadurch gekennzeichnet, dass** das mindestens eine Trägersubstrat (112; 210, 216; 710, 712, 714; 810, 812) Analyt-undurchlässig ist.

6. Implantierbarer Sensor (110) gemäß einem der vorhergehenden Ansprüche, **dadurch gekennzeichnet, dass** das mindestens eine Trägersubstrat (112; 210, 216; 710, 712, 714; 810, 812) ein Polymer, insbesondere ein isolierendes Polymer, insbesondere einen Polyester, aufweist.

7. Implantierbarer Sensor (110) gemäß einem der vorhergehenden Ansprüche, **dadurch gekennzeichnet, dass** mindestens eine Gegenelektrode (140; 742; 830) und mindestens eine Referenzelektrode (142; 738; 832) vorgesehen sind, welche als eine gemeinsame Elektrode (138) ausgebildet sind.

8. Implantierbarer Sensor (110) gemäß einem der vorhergehenden Ansprüche, **gekennzeichnet durch** eine Einführspitze (148) zum Einstechen des implantierbaren Sensors (110) in das Medium, insbesondere in ein Fettgewebe.

9. Implantierbarer Sensor (110) gemäß dem vorhergehenden Anspruch, **dadurch gekennzeichnet, dass** das mindestens eine isolierende Trägersubstrat (112) und/oder der implantierbare Sensor (110) eine Breite B und eine Höhe H aufweist, wobei das Verhältnis H/B oder dessen Kehrwert ein Aspektverhältnis k definiert, wobei das Aspektverhältnis k mindestens 0,3, vorzugsweise mindestens 0,5 und besonders bevorzugt mindestens 0,9 beträgt.

10. Implantierbarer Sensor (110) gemäß einem der vorhergehenden Ansprüche, **dadurch gekennzeichnet, dass** der Schichtaufbau ein gestufter Schichtaufbau ist, wobei mindestens zwei isolierende Trägersubstrate (112; 210, 216; 710, 712, 714; 810, 812) mindestens eine Stufe (220; 724, 728; 820) bilden.

11. Implantierbarer Sensor (110) gemäß dem vorhergehenden Anspruch, **dadurch gekennzeichnet, dass** mindestens eine Elektrode (134, 136; 238, 240, 242; 738, 740, 742; 830, 832, 836) in der Ebene der mindestens einen Stufe (220; 724, 728; 820) angeordnet ist und dass die mindestens eine in der Ebene der mindestens einen Stufe (220; 724, 728; 820) angeordnete Elektrode (134, 136; 238, 240, 242; 738, 740, 742; 830, 832, 836) und mindestens eine weitere Elektrode (134, 136; 238, 240, 242; 738, 740, 742; 830, 832, 836) parallele, gleichgerichtete Elektrodenflächen aufweisen.

12. Implantierbarer Sensor (110) gemäß einem der beiden vorhergehenden Ansprüche, **dadurch gekennzeichnet, dass** zwei Stufen (220; 724, 728; 820) mit in den zwei Ebenen der zwei Stufen (220; 724, 728; 820) angeordneten, gleichgerichteten Elektroden (134, 136; 238, 240, 242; 738, 740, 742; 830, 832, 836) vorgesehen sind.

13. Implantierbarer Sensor (110) gemäß einem der drei vorhergehenden Ansprüche, **dadurch gekennzeichnet, dass** zusätzlich mindestens eine weitere Elektrode (134, 136; 238, 240, 242; 738, 740, 742; 830, 832, 836) vorgesehen ist, welche auf einer der mindestens einen Stufe (220; 724, 728; 820) abgewandten Seite des mindestens einen Trägersubstrats (112; 210, 216; 710, 712, 714; 810, 812) angeordnet ist und mit ihrer Elektrodenfläche entgegengesetzt zur Stufe (220; 724, 728; 820) orientiert ist.

14. Implantierbarer Sensor (110) gemäß einem der vorhergehenden Ansprüche, **dadurch gekennzeichnet, dass** mindestens zwei Elektroden (134, 136; 238, 240, 242; 738, 740, 742; 830, 832, 836) auf gegenüberliegenden Seiten des mindestens einen Trägersubstrats (112; 210, 216; 710, 712, 714; 810, 812) angeordnet sind und entgegengesetzt gerichtete, dem Medium zugewandte Elektrodenflächen aufweisen.

15. Implantierbarer Sensor (110) gemäß einem der vorhergehenden Ansprüche, **gekennzeichnet durch** einen Back-to-Back-Aufbau, mit mindestens einem zwischen einer ersten Elektrodenkontaktschicht (114, 116; 212, 214; 222; 610, 612; 716, 718, 720; 814, 816, 822) und einer zweiten Elektrodenkontaktschicht (114, 116; 212, 214; 222; 610, 612; 716, 718, 720; 814, 816, 822) eingebettetem Trägersubstrat (112; 210, 216; 710, 712, 714; 810, 812), wobei auf der dem mindestens einen Trägersubstrat (112; 210, 216; 710, 712, 714; 810, 812) abgewandten Seite der ersten Elektrodenkontaktschicht (114, 116; 212, 214; 222; 610, 612; 716, 718, 720; 814, 816, 822) mindestens eine erste Elektrode (134, 136; 238, 240, 242; 738, 740, 742; 830, 832, 836) vorgesehen ist und wobei auf der dem Trägersubstrat (112; 210, 216; 710, 712, 714; 810, 812) abgewandten Seite der zweiten Elektrodenkontaktschicht (114, 116; 212, 214; 222; 610, 612; 716, 718, 720; 814, 816, 822) mindestens eine zweite Elektrode (134, . 136; 238, 240, 242; 738, 740, 742; 830, 832, 836) vorgesehen ist.

16. Vorrichtung (310) zur Ermittlung einer Konzentration mindestens eines Analyten in einem Medium, insbesondere einem Körpergewebe und/oder einer Körperflüssigkeit, umfassend mindestens einen implantierbaren Sensor (110) gemäß einem der vorhergehenden Ansprüche sowie weiterhin mindestens eine Spannungsmessvorrichtung (312) zur Messung einer Spannung zwischen mindestens einer Arbeitselektrode (144; 740; 836) und mindestens einer Referenzelektrode (142; 738; 832) des implantierbaren Sensors (110).

17. Vorrichtung (310) gemäß dem vorhergehenden Anspruch, umfassend weiterhin mindestens eine Strommessvorrichtung (314) zur Messung eines Stroms zwischen mindestens einer Gegenelektrode (140; 742; 830) und mindestens einer Arbeitselektrode (144; 740; 836) des implantierbaren Sensors (110).

18. Verfahren zur Herstellung eines implantierbaren Sensors (110) zur Ermittlung einer Analytkonzentration in einem Medium, insbesondere einem Körpergewebe und/oder einer Körperflüssigkeit, umfassend folgende Schritte:
- Ein Schichtaufbau wird erzeugt, wobei folgende Verfahrensschritte eingesetzt werden:
o Mindestens zwei Elektrodenkontaktschichten (114, 116; 212, 214; 222; 610, 612; 716, 718, 720; 814, 816, 822), insbesondere mindestens zwei Metallschichten, werden in mindestens zwei verschiedenen Schichtebenen auf mindestens eine mindestens ein isolierendes Material umfassende Trägerfolie aufgebracht;
o mindestens zwei Elektrodensysteme (130, 132; 232, 234, 236; 616, 618; 732, 734, 736; 826, 828) werden auf die mindestens zwei Elektrodenkontaktschichten (114, 116; 212, 214; 222; 610, 612; 716, 718, 720; 814; 816, 822) aufgebracht,
**dadurch gekennzeichnet, dass** die mindestens zwei Elektrodenkontaktschichten (114, 116; 212, 214; 222; 610, 612; 716, 718, 720; 814, 816, 822) großflächig auf die Trägerfolie aufgebracht werden, wobei mittels eines Präzisions-Schneideverfahrens der Schichtaufbau in Sensorstreifen geschnitten wird, derart, dass das mindestens eine isolierende Trägersubstrat (112; 210, 216; 710, 712, 714; 810, 812) eine Breite aufweist, wobei sich die mindestens zwei Elektroden (134, 136; 238, 240, 242; 738, 740, 742; 830, 832, 836) und/oder die mindestens zwei Elektrodenkontaktschichten (114, 116; 212, 214; 222; 610, 612; 716, 718, 720; 814, 816, 822) über die gesamte Breite des mindestens einen isolierenden Trägersubstrats (112; 210, 216; 710, 712, 714; 810, 812) erstrecken.

19. Verfahren gemäß dem vorhergehenden Anspruch, **dadurch gekennzeichnet, dass** mindestens eines der folgenden Verfahren eingesetzt wird:
- ein Laminierverfahren;
- ein Rolle-zu-Rolle-Folienverfahren;
- ein PVD-Verfahren und/oder ein CVD-Verfahren;
- ein nasschemisches Beschichtungsverfahren.

20. Verfahren gemäß einem der beiden vorhergehenden Ansprüche, **dadurch gekennzeichnet, dass** kein zusätzlicher Strukturierungsschritt der mindestens zwei Elektrodenkontaktschichten (114, 116; 212, 214; 222; 610, 612; 716, 718, 720; 814, 816, 822) erfolgt.

21. Verfahren gemäß einem der vorhergehenden Verfahrensansprüche, **dadurch gekennzeichnet, dass** mindestens eine erste Trägerfolie beidseitig mit Elektrodenkontaktschichten (114, 116; 212, 214; 222; 610, 612; 716, 718, 720; 814, 816, 822) umgeben, insbesondere beschichtet, wird, wobei auf eine der beiden Elektrodenkontaktschichten (114, 116; 212, 214; 222; 610, 612; 716, 718, 720; 814, 816, 822) auf der der Trägerfolie abgewandten Seite mindestens ein erstes Elektrodensystem (130, 132; 232, 234, 236; 616, 618; 732, 734, 736; 826, 828) aufgebracht wird und wobei auf die andere der beiden Elektrodenkontaktschichten (114, 116; 212, 214; 222; 610, 612; 716, 718, 720; 814, 816, 822) auf der der Trägerfolie abgewandten Seite mindestens ein zweites Elektrodensystem (130, 132; 232, 234, 236; 616, 618; 732, 734, 736; 826, 828) aufgebracht wird.

22. Verfahren gemäß dem vorhergehenden Anspruch, wobei zusätzlich eine zweite Trägerfolie einseitig mit einer dritten Elektrodenkontaktschicht (114, 116; 212, 214; 222; 610, 612; 716, 718, 720; 814, 816, 822) in Kontakt gebracht, insbesondere beschichtet, wird, wobei auf die dritte Elektrodenkontaktschicht (114, 116; 212, 214; 222; 610, 612; 716, 718, 720; 814, 816, 822) ein drittes Elektrodensystem (130, 132; 232, 234, 236; 616, 618; 732, 734, 736; 826, 828) aufgebracht wird, wobei die zweite Trägerfolie mit der der dritten Elektrodenkontaktschicht (114, 116; 212, 214; 222; 610, 612; 716, 718, 720; 814, 816, 822) abgewandten Seite auf die beidseitig mit Elektrodenkontaktschichten (114, 116; 212, 214; 222; 610, 612; 716, 718, 720; 814, 816, 822) umgebene erste Trägerfolie aufgebracht wird, dergestalt, dass mindestens eine Stufe (220; 724, 728; 820) entsteht.

23. Verfahren gemäß einem der vorhergehenden Verfahrensansprüche, **dadurch gekennzeichnet, dass** zusätzlich mindestens eine Isolatorschicht (118, 120), insbesondere mindestens eine selbstklebende Folienschicht, aufgebracht wird.

## Claims

1. An implantable sensor (110) for determining a concentration of at least one analyte in a medium, in particular a bodily tissue and/or a bodily fluid, the implantable sensor (110) comprising a multilayer structure with at least one insulating carrier substrate (112; 210, 216; 710, 712, 714; 810, 812) and at least two electrodes (134, 136; 238, 240, 242; 738; 740, 742; 830, 832, 836) arranged in at least two different layer planes of the implantable sensor (110), electrically isolated from one another by the at least one insulating carrier substrate (112; 210, 216; 710, 712, 714; 810, 812) and having electrode surfaces, the electrode surfaces in the implanted sensor (110) facing the medium and, directly or via an analyte-permeable membrane layer (146), being extensively and substantially uniformly in contact with the medium, the implantable sensor (110) furthermore comprising electrode contact layers (114, 116; 212, 214; 222; 610, 612; 716, 718, 720; 814, 816, 822) electrically contacting the at least two electrodes (134, 136; 238, 240, 242; 738, 740, 742; 830, 832, 836), **characterised in that** the at least one insulating carrier substrate (112; 210, 216; 710, 712, 714; 810, 812) comprises a width such that the at least two electrodes (134, 136; 238, 240, 242; 738, 740, 742; 830, 832, 836) and/or the at least two electrode contact layers (114, 116; 212, 214; 222; 610, 612; 716, 718, 720; 814, 816, 822) extend over the entire width of the at least one insulating carrier substrate (112; 210, 216; 710, 712, 714; 810, 812).

2. An implantable sensor according to the preceding claim, **characterised in that** the at least two electrodes (134, 136; 238, 240, 242; 738, 740, 742; 830, 832, 836) comprise at least one working electrode (144; 740; 836) and at least one further electrode (134, 136; 238, 240, 242; 738, 742; 830, 832), in particular at least one counter-electrode (140; 742; 830) and/or at least one reference electrode (142; 738; 832), the at least one working electrode (144; 740; 836) and the at least one further electrode (134, 136; 238, 240, 242; 738, 742; 830, 832) being arranged in different layer planes of the multilayer structure.

3. An implantable sensor (110) according to one of the two preceding claims, **characterised in that** the at least two electrode contact layers (114, 116; 212, 214; 222; 610, 612; 716, 718, 720; 814, 816, 822) are screened, preferably electrically insulated, at least partially, preferably completely, by at least one insulator layer (118, 120), preferably by at least one self-adhesive film layer, against the medium, in particular the bodily tissue and/or the bodily fluid.

4. An implantable sensor (110) according to any one of the preceding claims, **characterised in that** the at least one membrane layer (146) comprises a polyurethane.

5. An implantable sensor (110) according to any one of the preceding claims, **characterised in that** the at least one carrier substrate (112; 210, 216; 710, 712, 714; 810, 812) is impermeable to analytes.

6. An implantable sensor (110) according to any one of the preceding claims, **characterised in that** the at least one carrier substrate (112; 210, 216; 710, 712, 714; 810, 812) comprises a polymer, in particular an insulating polymer, in particular a polyester.

7. An implantable sensor (110) according to any one of the preceding claims, **characterised in that** at least one counter-electrode (140; 742; 830) and at least one reference electrode (142; 738; 832) are provided, which take the form of a joint electrode (138).

8. An implantable sensor (110) according to any one of the preceding claims, **characterised by** a insertion tip (148) for pushing the implantable sensor (110) into the medium, in particular into fatty tissue.

9. An implantable sensor (110) according to the preceding claim, **characterised in that** the at least one insulating carrier substrate (112) and/or the implantable sensor (110) has a width B and a height H, the ratio H/B or the reciprocal thereof defining an aspect ratio k, the aspect ratio k amounting to at least 0.3, preferably at least 0.5 and particularly preferably at least 0.9.

10. An implantable sensor (110) according to any one of the preceding claims, **characterised in that** the multilayer structure is a stepped multilayer structure, at least two insulating carrier substrates (112; 210, 216; 710, 712, 714; 810, 812) forming at least one step (220; 724, 728; 820).

11. An implantable sensor (110) according to the preceding claim, **characterised in that** at least one electrode (134, 136; 238, 240, 242; 738, 740, 742; 830, 832, 836) is arranged in the plane of the at least one step (220; 724, 728; 820) and that the at least one electrode (134, 136; 238, 240, 242; 738, 740, 742; 830, 832, 836) arranged in the plane of the at least one step (220; 724, 728; 820) and at least one further electrode (134, 136; 238, 240, 242; 738, 740, 742; 830, 832, 836) comprise parallel, identically oriented electrode surfaces.

12. An implantable sensor (110) according to one of the two preceding claims, **characterised in that** two steps (220; 724, 728; 820) are provided with identically oriented electrodes (134, 136; 238, 240, 242; 738, 740, 742; 830, 832, 836) arranged in the two planes of the two steps (220; 724, 728; 820).

13. An implantable sensor (110) according to one of the three preceding claims, **characterised in that,** in addition, at least one further electrode (134, 136; 238, 240, 242; 738, 740, 742; 830, 832, 836) is provided, which is arranged on a side of the at least one carrier substrate (112; 210, 216; 710, 712, 714; 810, 812) remote from the at least one step (220; 724, 728; 820) and is oriented with its electrode surface in the opposite direction from the step (220; 724, 728; 820).

14. An implantable sensor (110) according to any one of the preceding claims, **characterised in that** at least two electrodes (134, 136; 238, 240, 242; 738, 740, 742; 830, 832, 836) are arranged on opposing sides of the at least one carrier substrate (112; 210, 216; 710, 712, 714; 810, 812) and comprise opposingly directed electrode surfaces facing the medium.

15. An implantable sensor (110) according to any one of the preceding claims, **characterised by** a back-to-back structure, having at least one carrier substrate (112; 210, 216; 710, 712, 714; 810, 812) embedded between a first electrode contact layer (114, 116; 212, 214; 222; 610, 612; 716, 718, 720; 814, 816, 822) and a second electrode contact layer (114, 116; 212, 214; 222; 610, 612; 716, 718, 720; 814, 816, 822), at least one first electrode (134, 136; 238, 240, 242; 738, 740, 742; 830, 832, 836) being provided on the side of the first electrode contact layer (114, 116; 212, 214; 222; 610, 612; 716, 718, 720; 814, 816, 822) remote from the at least one carrier substrate (112; 210, 216; 710, 712, 714; 810, 812) and at least one second electrode (134, 136; 238, 240, 242; 738, 740, 742; 830, 832, 836) being provided on the side of the second electrode contact layer (114, 116; 212, 214; 222; 610, 612; 716, 718, 720; 814, 816, 822) remote from the carrier substrate (112; 210, 216; 710, 712, 714; 810, 812).

16. A device (310) for determining a concentration of at least one analyte in a medium, in particular a bodily tissue and/or a bodily fluid, comprising at least one implantable sensor (110) according to any one of the preceding claims and further comprising at least one voltage measuring device (312) for measuring a voltage between at least one working electrode (144; 740; 836) and at least one reference electrode (142; 738; 832) of the implantable sensor (110).

17. A device (310) according to the preceding claim, further comprising at least one current measuring device (314) for measuring a current between at least one counter-electrode (140; 742; 830) and at least one working electrode (144; 740; 836) of the implantable sensor (110).

18. A method for producing an implantable sensor (110) for determining an analyte concentration in a medium, in particular a bodily tissue and/or a bodily fluid, having the following steps:
- a multilayer structure is produced, the following method steps being used:
o at least two electrode contact layers (114, 116; 212, 214; 222; 610, 612; 716, 718, 720; 814, 816, 822), in particular at least two metal layers, are applied in at least two different layer planes onto at least one carrier film comprising at least one insulating material;
o at least two electrode systems (130, 132; 232, 234, 236; 616, 618; 732, 734, 736; 826, 828) are applied onto the at least two electrode contact layers (114, 116; 212, 214; 222; 610, 612; 716, 718, 720; 814, 816, 822),
**characterised in that** the at least two electrode contact layers (114, 116; 212, 214; 222; 610, 612; 716, 718, 720; 814, 816, 822) are applied extensively onto the carrier film, the multilayer structure being cut into sensor strips by means of a precision cutting method, in such a way that the at least one insulating carrier substrate (112; 210, 216; 710, 712, 714; 810, 812) comprises a width such that the at least two electrodes (134, 136; 238, 240, 242; 738, 740, 742; 830, 832, 836) and/or the at least two electrode contact layers (114, 116; 212, 214; 222; 610, 612; 716, 718, 720; 814, 816, 822) extend over the entire width of the at least one insulating carrier substrate (112; 210, 216; 710, 712, 714; 810, 812).

19. A method according to the preceding claim, **characterised in that** at least one of the following methods is used:
- a laminating method;
- a roll-to-roll film method;
- a PVD method and/or a CVD method;
- a wet chemical coating method.

20. A method according to one of the two preceding claims, **characterised in that** no additional structuring step of the at least two electrode contact layers (114, 116; 212, 214; 222; 610, 612; 716, 718, 720; 814; 816, 822) is performed.

21. A method according to one of the preceding method claims, **characterised in that** at least one first carrier film is surrounded, in particular coated, on both sides with electrode contact layers (114, 116; 212, 214; 222; 610, 612; 716, 718, 720; 814, 816, 822), at least one first electrode system (130, 132; 232, 234, 236; 616, 618; 732, 734, 736; 826, 828) being applied onto one of the two electrode contact layers (114, 116; 212, 214; 222; 610, 612; 716, 718, 720; 814, 816, 822) on the side remote from the carrier film and at least one second electrode system (130, 132; 232, 234, 236; 616, 618; 732, 734, 736; 826, 828) being applied onto the other of the two electrode contact layers (114, 116; 212, 214; 222; 610, 612; 716, 718, 720; 814, 816, 822) on the side remote from the carrier film.

22. A method according to the preceding claim, a second carrier film additionally being brought into contact, in particular coated, on one side with a third electrode contact layer (114, 116; 212, 214; 222; 610, 612; 716, 718, 720; 814, 816, 822), a third electrode system (130, 132; 232, 234, 236; 616, 618; 732, 734, 736; 826, 828) being applied onto the third electrode contact layer (114, 116; 212, 214; 222; 610, 612; 716, 718, 720; 814, 816, 822), the second carrier film being applied with the side remote from the third electrode contact layer (114, 116; 212, 214; 222; 610, 612; 716, 718, 720; 814, 816, 822) onto the first carrier film surrounded on both sides with electrode contact layers (114, 116; 212, 214; 222; 610, 612; 716, 718, 720; 814, 816, 822), in such a manner that at least one step (220; 724, 728; 820) is obtained.

23. A method according to any one of the preceding method claims, **characterised in that** at least one insulator layer (118, 120), in particular at least one self-adhesive film layer, is additionally applied.

## Revendications

1. Capteur implantable (110) destiné à déterminer une concentration d'au moins un analyte dans un milieu, en particulier un tissu corporel et/ou un liquide corporel, le capteur implantable (110) présentant une structure de couches avec au moins un substrat de support isolant (112; 210, 216; 710, 712, 714; 810, 812) et au moins deux électrodes (134, 136; 238, 240, 242; 738; 740, 742; 830, 832, 836) comportant des surfaces électrodiques, lesquelles électrodes sont disposées dans au moins deux plans de couches différents du capteur implantable (110) et séparées électriquement par l'au moins un substrat de support isolant (112; 210, 216; 710, 712, 714; 810, 812), les surfaces électrodiques étant, lorsque le capteur (110) est implanté, tournées vers le milieu et étant en contact, directement ou par l'intermédiaire d'une couche membraneuse (146) perméable à l'analyte, avec le milieu sur une grande surface et de manière sensiblement uniforme, le capteur implantable (110) comportant également des couches de contact électrodiques (114, 116; 212, 214; 222; 610, 612; 716, 718, 720; 814, 816, 822) qui contactent électriquement les au moins deux électrodes (134, 136; 238, 240, 242; 738; 740, 742; 830, 832, 836), **caractérisé en ce que** l'au moins un substrat de support isolant (112; 210, 216; 710, 712, 714; 810, 812) présente une largeur, les au moins deux électrodes (134, 136; 238, 240, 242; 738; 740, 742; 830, 832, 836) et/ou les au moins deux couches de contact électrodiques (114, 116; 212, 214; 222; 610, 612; 716, 718, 720; 814, 816, 822) s'étendant sur toute la largeur de l'au moins un substrat de support isolant (112; 210, 216; 710, 712, 714; 810, 812).

2. Capteur implantable selon la revendication précédente, **caractérisé en ce que** les au moins deux électrodes (134, 136; 238, 240, 242; 738; 740, 742; 830, 832, 836) comprennent au moins une électrode de travail (144; 740; 836) et au moins une autre électrode (134, 136; 238, 240, 242; 738; 742; 830, 832), en particulier au moins une contre-électrode (140; 742; 830) et/ou au moins une électrode de référence (142; 738; 832), l'au moins une électrode de travail (144; 740; 836) et l'au moins une autre électrode (134, 136; 238, 240, 242; 738; 742; 830, 832,) étant situées dans différents plans de couches de la structure de couches.

3. Capteur implantable (110) selon l'une des deux revendications précédentes, **caractérisé en ce que** les au moins deux couches de contact électrodiques (114, 116; 212, 214; 222; 610, 612; 716, 718, 720; 814, 816, 822) sont recouvertes, et de préférence isolées électriquement, au moins en partie, et de préférence entièrement, par rapport au milieu, en particulier au tissu corporel et/ou au liquide corporel, par au moins une couche d'isolateur (118, 120), et de préférence par au moins une couche pelliculaire autocollante.

4. Capteur implantable (110) selon l'une des revendications précédentes, **caractérisé en ce que** l'au moins une couche membraneuse (146) comporte un polyuréthane.

5. Capteur implantable (110) selon l'une des revendications précédentes, **caractérisé en ce que** l'au moins un substrat de support (112; 210, 216; 710, 712, 714; 810, 812) est perméable à l'analyte.

6. Capteur implantable (110) selon l'une des revendications précédentes, **caractérisé en ce que** l'au moins un substrat de support (112; 210, 216; 710, 712, 714; 810, 812) est un polymère, en particulier un polymère isolant, en particulier un polyester.

7. Capteur implantable (110) selon l'une des revendications précédentes, **caractérisé en ce que** sont prévues au moins une contre-électrode (140; 742; 830) et au moins une électrode de référence (142; 738; 832), lesquelles se présentent sous la forme d'une électrode commune (138).

8. Capteur implantable (110) selon l'une des revendications précédentes, **caractérisé par** une pointe d'insertion (148) pour enfoncer le capteur implantable (110) dans le milieu, en particulier dans un tissu adipeux.

9. Capteur implantable (110) selon la revendication précédente, **caractérisé en ce que** l'au moins un substrat de support isolant (112) et/ou le capteur implantable (110) ont une largeur B et une hauteur H, le rapport H/B ou son inverse définissant un rapport d'aspect k, le rapport d'aspect k étant d'au moins 0,3, de préférence d'au moins 0,5 et, particulièrement préférentiellement, d'au moins 0,9.

10. Capteur implantable (110) selon l'une des revendications précédentes, **caractérisé en ce que** la structure de couches est une structure de couches étagée, au moins deux substrats de supports isolants (112; 210, 216; 710, 712, 714; 810, 812) constituant au moins un étage (220; 724; 728; 820).

11. Capteur implantable (110) selon la revendication précédente, **caractérisé en ce qu'**au moins une électrode (134, 136; 238, 240, 242; 738; 740, 742; 830, 832, 836) est située dans le plan de l'au moins un étage (220; 724; 728; 820) et **en ce que** l'au moins une électrode (134, 136; 238, 240, 242; 738; 740, 742; 830, 832, 836) située dans le plan de l'au moins un étage (220; 724, 728; 820) et au moins une autre électrode (134, 136; 238, 240, 242; 738; 740, 742; 830, 832, 836) présentent des surfaces électrodiques parallèles et orientées dans le même sens.

12. Capteur implantable (110) selon l'une des deux revendications précédentes, **caractérisé en ce que** sont prévus deux étages (220; 724, 728; 820) avec des électrodes (134, 136; 238, 240, 242; 738; 740, 742; 830, 832, 836) orientées dans le même sens et situées dans les deux plans des deux étages (220; 724, 728; 820).

13. Capteur implantable (110) selon l'une des trois revendications précédentes, **caractérisé en ce qu'**est additionnellement prévue au moins une autre électrode (134, 136; 238, 240, 242; 738, 740, 742; 830, 832, 836), laquelle est située sur un côté de l'au moins un substrat de support (112; 210, 216; 710, 712, 714; 810, 812) opposé à l'au moins un plan (220; 724, 728; 820) et dont la surface électrodique est orientée de manière opposée à l'étage (220; 724, 728; 820).

14. Capteur implantable (110) selon l'une des revendications précédentes, **caractérisé en ce qu'**au moins deux électrodes (134, 136; 238, 240, 242; 738, 740, 742; 830, 832, 836) sont situées sur des côtés opposés de l'au moins un substrat de support (112; 210, 216; 710, 712, 714; 810, 812) et présentent des surfaces électrodiques dirigées en sens inverse et tournées vers le milieu.

15. Capteur implantable (110) selon l'une des revendications précédentes, **caractérisé par** une structure dos à dos, avec au moins un substrat de support (112; 210, 216; 710, 712, 714; 810, 812) inséré entre une première couche de contact électrodique (114, 116; 212, 214; 222; 610, 612; 716, 718, 720; 814, 816, 822) et une deuxième couche de contact électrodique (114, 116; 212, 214; 222; 610, 612; 716, 718, 720; 814, 816, 822), au moins une première électrode (134, 136; 238, 240, 242; 738, 740, 742; 830, 832, 836) étant prévue sur le côté de la première couche de contact électrodique (114, 116; 212, 214; 222; 610, 612; 716, 718, 720; 814, 816, 822) opposée à l'au moins un substrat de support (112; 210, 216; 710, 712, 714; 810, 812) et au moins une deuxième électrode (134, 136; 238, 240, 242; 738, 740, 742; 830, 832, 836) étant prévue sur le côté de la deuxième couche de contact électrodique (114, 116; 212, 214; 222; 610, 612; 716, 718, 720; 814, 816, 822) opposée au substrat de support (112; 210, 216; 710, 712, 714; 810,812).

16. Dispositif (310) destiné à déterminer une concentration d'au moins un analyte dans un milieu, en particulier un tissu corporel et/ou un liquide corporel, comprenant au moins un capteur implantable (110) selon l'une des revendications précédentes et, en outre, au moins un dispositif de mesure de tension (312) pour mesurer une tension entre au moins une électrode de travail (144; 740; 836) et au moins une électrode de référence (142; 738; 832) du capteur implantable (110).

17. Dispositif (310) selon la revendication précédente, comprenant en outre au moins un dispositif de mesure de courant (314) pour mesurer un courant entre au moins une contre-électrode (140; 742; 830) et au moins une électrode de travail (144; 740; 836) du capteur implantable (110).

18. Procédé de fabrication d'un capteur implantable (110) destiné à déterminer une concentration d'analyte dans un milieu, en particulier un tissu corporel et/ou un liquide corporel, comprenant les étapes suivantes:
- production d'une structure de couches, les étapes suivantes étant mises en oeuvre:
■ au moins deux couches de contact électrodiques (114, 116; 212, 214; 222; 610, 612; 716, 718, 720; 814, 816, 822), en particulier au moins deux couches métalliques, sont appliquées dans au moins deux plans de couches différents sur au moins une feuille de support comprenant une matière isolante;
■ au moins deux systèmes d'électrodes (130, 132; 232, 234, 236; 616, 618; 732, 734, 736; 826, 828) sont appliqués sur les au moins deux couches de contact électrodiques (114, 116; 212, 214; 222; 610, 612; 716, 718, 720; 814, 816, 822),
**caractérisé en ce que** les au moins deux couches de contact électrodiques (114, 116; 212, 214; 222; 610, 612; 716, 718, 720; 814, 816, 822) sont appliquées sur une grande surface sur la feuille de support, la structure de couches étant coupée en bandes de capteurs au moyen d'un procédé de découpe de précision de manière telle que l'au moins un substrat de support isolant (112; 210, 216; 710, 712, 714; 810, 812) présente une largeur, les au moins deux électrodes (134, 136; 238, 240, 242; 738; 740, 742; 830, 832, 836) et/ou les au moins deux couches de contact électrodiques (114, 116; 212, 214; 222; 610, 612; 716, 718, 720; 814, 816, 822) s'étendant sur toute la largeur de l'au moins un substrat de support isolant (112; 210, 216; 710, 712, 714; 810, 812).

19. Procédé selon la revendication précédente, **caractérisé en ce qu'**au moins l'un des procédés suivants est mis en oeuvre:
- un procédé de laminage,
- un procédé flexo bobine/bobine,
- un procédé PVD et/ou un procédé CVD,
- un procédé de revêtement chimique par voie humide.

20. Procédé selon l'une des deux revendications précédentes, **caractérisé en ce que** n'est effectuée aucune autre étape de structuration supplémentaire des au moins deux couches de contact électrodiques (114, 116; 212, 214; 222; 610, 612; 716, 718, 720; 814, 816, 822).

21. Procédé selon l'une quelconque des revendications de procédés précédentes, **caractérisé en ce qu'**au moins une première feuille de support est entourée, et plus particulièrement revêtue de couches de contact électrodiques (114, 116; 212, 214; 222; 610, 612; 716, 718, 720; 814, 816, 822) des deux côtés, au moins un premier système d'électrodes (130, 132; 232, 234, 236; 616, 618; 732, 734, 736; 826, 828) étant appliqué sur l'une des deux couches de contact électrodiques (114, 116; 212, 214; 222; 610, 612; 716, 718, 720; 814, 816, 822) sur le côté opposé à la feuille de support et au moins un deuxième système d'électrodes (130, 132; 232, 234, 236; 616, 618; 732, 734, 736; 826, 828) étant appliqué sur l'autre des deux couches de contact électrodiques (114, 116; 212, 214; 222; 610, 612; 716, 718, 720; 814, 816, 822) sur le côté opposé à la feuille de support.

22. Procédé selon la revendication précédente, une deuxième feuille de support étant additionnellement mise en contact, et plus particulièrement revêtue, d'un côté, avec une troisième couche de contact électrodique (114, 116; 212, 214; 222; 610, 612; 716, 718, 720; 814, 816, 822), un troisième système d'électrodes (130, 132; 232, 234, 236; 616, 618; 732, 734, 736; 826, 828) étant appliqué sur la troisième couche de contact électrodique (114, 116; 212, 214; 222; 610, 612; 716, 718, 720; 814, 816, 822), la deuxième feuille de support étant appliquée, en sa face opposée à la troisième couche de contact électrodique (114, 116; 212, 214; 222; 610, 612; 716, 718, 720; 814, 816, 822), sur la première feuille de support, entourée de couches de contact électrodiques (114, 116; 212, 214; 222; 610, 612; 716, 718, 720; 814, 816, 822) des deux côtés, de manière telle qu'il se forme au moins un étage (220; 724, 728; 820).

23. Procédé selon l'une quelconque des revendications de procédés précédentes, **caractérisé en ce qu'**est appliquée, additionnellement, au moins une couche d'isolateur (118, 120), en particulier au moins une couche pelliculaire autocollante.
